# EUROPEAN PATENT SPECIFICATION

(11) **EP 0 453 871 B1**
(45) Date of publication and mention of the grant of the patent: **07.02.1996**
(21) Application number: 91105664.6
(22) Date of filing: 10.04.1991
(51) Int. Cl.: C07F 9/6521, C08K 5/52, C07F 9/6558, C07F 9/6574, C07D 251/70

(54) **Salts of triazine derivatives with oxygenated acids of phosphorus**
Triazinderivatsalze mit Oxasäuren des Phosphors
Sels de dérivés triaziniques avec des acides oxygénés du phosphore

(30) Priority: 11.04.1990 IT 2000790
(43) Date of publication of application: 30.10.1991
(73) Proprietor: MINISTERO DELL' UNIVERSITA' E DELLA RICERCA SCIENTIFICA E TECNOLOGICA, I-00196 Roma (IT)
(72) Inventor: Cipolli, Roberto, I-28100 Novara (IT); Oriani, Roberto, I-20137 Milan (IT); Nucida, Gilberto, I-20098 San Giuliano Milanese, Milan (IT); Masarati, Enrico, I-29010 Castelnuovo (IT)
(74) Representative: Weinhold, Peter, Dr.

(56) References cited:
- EP-A- 0 055 216
- US-A- 3 296 265
- US-A- 4 154 930
- 'Flame Retardancy of Polymeric Materials', Vol 2, Pages 221-223, Marcel Dekker, Inc., New York.
- 's-Triazines and derivatives', page 338, Interscience Publishers, Inc., New York, 1959.

## Description

The present invention relates to salts of triazine derivatives with oxygenated acids of phosphorus, which may be obtained by salifying derivatives of 2,4,6-triamino-1,3,5-triazine with a phosphorus-containing acid.

These salts are capable of endowing thermoplastic polymers or polymers with elastomeric properties, particularly olefinic (co)polymers with highly satisfactory self-extinguishing (flame-retardant) characteristics.

US-A-4 154 930 discloses pentate salts of melamine and ammelide useful as flame retardants for polymers. US-A-3 296 265 discloses dialkyl phosphonate salts useful as flame proofing agents for synthetic polymers, cellulosic fibres and textiles as well as antistatic agents.

In particular, the present invention provides salts of general formula (I): 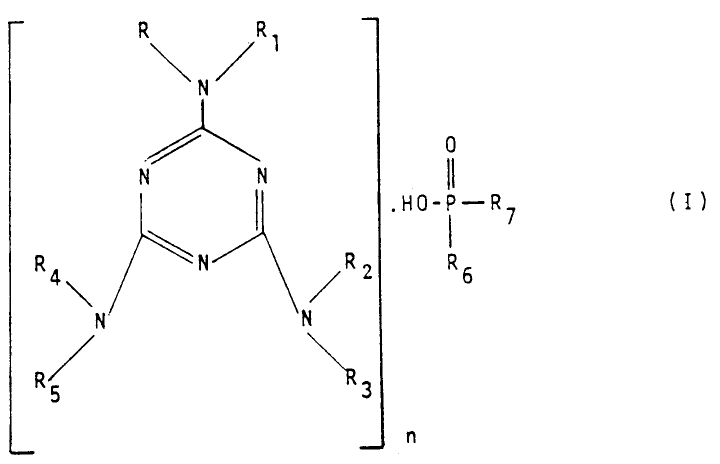
wherein:
at least one of the groups R and R₁ to R₅ is selected from ⁅CₘH₂ₘ⁆O-R₈ and 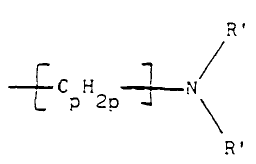
wherein

- m: = an integer of from 2 to 8, preferably from 2 to 4;
- p: = an integer of from 2 to 6, preferably from 2 to 4;
- R₈: = H; (C₁-C₈) and preferably (C₁-C₄) alkyl; (C₂-C₆) and preferably (C₂-C₄) alkenyl; ⁅C_{q}H_{2q}⁆O-R₉,
q being an integer of from 1 to 4 (e.g. 1 or 2) and
R₉ being hydrogen or (C₁-C₄)alkyl (e.g.methyl,ethyl); (C₆-C₁₂) and preferably (C₆-C₈) cycloalkyl or (C₆-C₁₂) alkylcycloalkyl;
the groups R', the same or different from each other, are selected from H; (C₁-C₈) and preferably (C₁-C₄) alkyl; (C₂-C₆) and preferably (C₂-C₄) alkenyl; (C₆-C₁₂) and preferably (C₆-C₈) cycloalkyl or (C₆-C₁₂) alkylcycloalkyl; and
(C₁-C₄) hydroxyalkyl, provided that R₆ and R₇, specified hereinafter, are different from H and OH;or the moiety N(R')₂ is replaced by an N-heterocyclic radical which optionally contains another heteroatom (preferably selected from 0, S and N) and is bound to the alkyl chain through the nitrogen atom;
or in general formula (I) at least one of the groups NRR₁, NR₂R₃ and NR₄R₅ is replaced by an N-heterocyclic radical which optionally contains another heteroatom (preferably selected from O, S and N)and is bound to the triazine ring through the nitrogen atom;
the remaining radicals R and R₁ to R₅, the same or different from each other, have the above meanings or are selected from H; (C₁-C₁₈) and preferably (C₁-C₈) alkyl; (C₂-C₈) and preferably (C₂-C₄) alkenyl; (C₆-C₁₆) and preferably (C₆-C₈) cycloalkyl or (C₆-C₁₆) alkylcycloalkyl, optionally substituted by a hydroxyl or (C₁-C₄) hydroxyalkyl group (e.g. hydroxymethyl or hydroxyethyl);
- n: is a number from 0.5 to 6, in particular from 0.5 to 5 and most preferred from 0.5 to 3;
- R₆: is selected from H; OH; (C₁-C₈) and preferably (C₁-C₄) alkoxy; aryloxy (preferably C₆-C₁₀), optionally substituted by a(C₁-C₈) alkyl group (e.g. methyl or ethyl); aralkyl (preferably C₇-C₁₄), optionally substituted by a (C₁-C₄) alkyl group; (C₁-C₄) alkyl, optionally substituted by a carboxylic group; and aryl (preferably C₆-C₁₀);
- R₇: is selected from H; OH; (C₁-C₈) and preferably (C₁-C₄) alkoxy; aryloxy (preferably C₆-C₁₀); (C₁-C₄) alkyl; aryl (preferably C₆-C₁₀); a group of formula

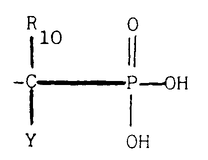

wherein R₁₀ is hydrogen or (C₁-C₁₂) and preferably (C₁-C₆) alkyl and Y is OH or R₁₀;
a group of formula 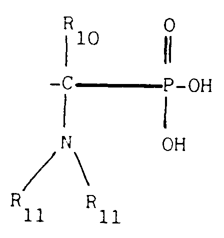
wherein R₁₀ is as defined above and the groups
R₁₁, the same or different from each other, represent hydrogen or (C₁-C₄) alkyl (e.g. methyl or ethyl) or the moiety N(R₁₁)₂ is replaced by an N-heterocyclic radical which optionally contains another heteroatom (preferably selected from O, S and N) and is bound to the carbon atom through the nitrogen atom;
a group of formula 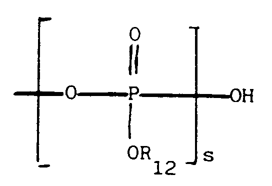
wherein R₁₂ is hydrogen or (C₁-C₈) and preferably (C₁-C₄) alkyl and s is an integer of from 1 to 3 (e.g. 1 or 2);
a group of formula 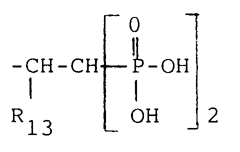
wherein R₁₃ is hydrogen or hydroxy;
a group of formula 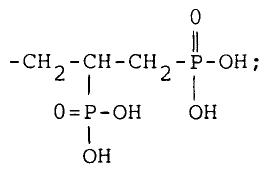
a group of formula 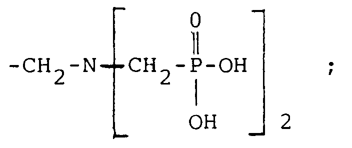
a group of formula 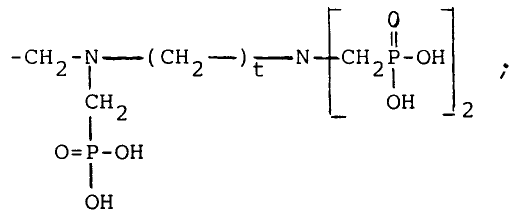
and a group of formula 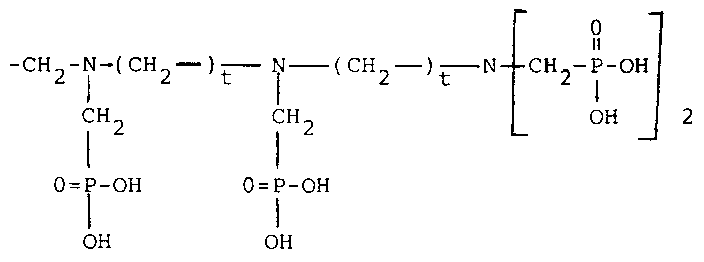
wherein t is an integer of from 2 to 6 (e.g. from 2 to 4); or R₆ and R₇ together form a cyclic structure of one of the following formulae: 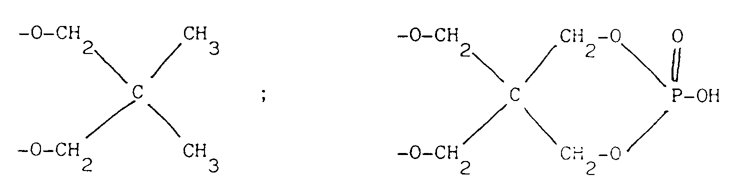
Examples of radicals R and R₁ to R₅ in general formula (I) are:
methyl; ethyl; propyl; isopropyl; n-butyl; isobutyl; tert.-butyl; n-pentyl; isopentyl; n-hexyl; tert.-hexyl, n-octyl; tert.-octyl; decyl; dodecyl; octadecyl; ethenyl; propenyl; butenyl; isobutenyl; hexenyl; octenyl; cyclohexyl; propylcyclohexyl; butylcyclohexyl; decylcyclohexyl; hydroxycyclohexyl; hydroxyethylcyclohexyl; 2-hydroxyethyl; 2-hydroxypropyl; 3-hydroxypropyl; 3-hydroxybutyl; 4-hydroxybutyl; 3-hydroxypentyl; 5-hydroxypentyl; 6-hydroxyhexyl; 3-hydroxy-2,5-dimethylhexyl; 7-hydroxyheptyl; 7-hydroxyoctyl; 2-methoxyethyl; 2-methoxypropyl; 3-methoxypropyl; 4-methoxybutyl; 6-methoxyhexyl; 7-methoxyheptyl; 7-methoxyoctyl; 2-ethoxyethyl; 3-ethoxypropyl; 4-ethoxybutyl; 3-propoxypropyl; 3-butoxypropyl; 4-butoxybutyl; 4-isobutoxybutyl; 5-propoxypentyl; 2-cyclohexyloxyethyl; 2-ethenyloxyethyl; 2-(N,N-dimethylamino)ethyl; 3-(N,N-dimethylamino)propyl; 4-(N,N-dimethylamino)butyl; 5-(N,N-dimethylamino)pentyl; 4-(N,N-diethylamino)butyl; 5-(N,N-diethylamino)pentyl; 5-(N,N-diisopropylamino)pentyl; 3-(N -ethylamino)propyl; 4-(N-methylamino)butyl; 4-(N,N-dipropylamino)hexyl; 4-(N,N-dipropylamino)butyl; 2-(N,N-diisopropylamino)ethyl; 6-(N-hexenylamino)hexyl; 2-(N-ethenylamino)ethyl; 2-(N-cyclohexylamino)ethyl; 2-(N-2-hydroxyethylamino)ethyl; 2-(2-hydroxyethoxy)ethyl; 2-(2-methoxyethoxy)ethyl; 6-(N-propylamino)hexyl; etc.

Examples of heterocyclic radicals which may replace the groups NRR₁, NR₂R₃ and NR₄R₅ in general formula (I) are aziridinyl; pyrrolidinyl; piperidinyl; morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl; 2-ethylpiperazinyl; 2,5-diethylpiperazinyl, etc. Particularly preferred examples are morpholinyl, thiomorpholinyl and piperazinyl.

Examples of heterocyclic radicals which may replace the moiety N(R')₂ are
aziridinyl, pyrrolidinyl, piperidinyl; morpholinyl; thiomorpholinyl; piperazinyl; 4-methylpiperazinyl; 4-ethylpiperazinyl; etc. Particularly preferred are morpholinyl, thiomorpholinyl and piperazinyl.

Examples of suitable phorphorus-containing acids are hypophosphorous acid; phosphorous acid; phosphoric acid; pyrophosphoric acid; tripolyphosphoric acid; ethane-1,1,2-triphosphonic acid; 2-hydroxyethane-1,1,2-triphosphonic acid; propane-1,2,3-triphosphonic acid; isopropylphosphoric acid; n-butylphosphoric acid; di-n-butylphosphoric acid; diisopropylphosphoric acid; di-n-pentylphosphoric acid; isooctylphosphoric acid; ethylphosphoric acid; hexylphosphoric acid; 2-ethylhexylphosphoric acid; methylphosphonic acid; ethylphosphonic acid; n-propylphosphonic acid; n-butylphosphonic acid; aminomethylphosphonic acid; phenylphosphoric acid; phenylphosphonic acid; phenylphosphinic acid; di-n-butylpyrophosphoric acid; di(2-ethylhexyl)pyrophosphoric acid; octylphenylphosphoric acid; 2-methylbenzylphosphonic acid; 1-aminoethane-1,1-diphosphonic acid; 1-hydroxyethane-1,1-diphosphonic acid; 1-hydroxydodecane-1,1-diphosphonic acid; 1-(N-methylamino)ethane-1,1-diphosphonic acid; N,N-dimethylaminomethane-1,1-diphosphonic acid; N-butylaminomethane-1,1-disphosphonic acid; phosphonoacetic acid; 2-phosphonopropionic acid; 3-phosphonopropionic acid; 2-phosphonobutyrric acid; 4-phosphonobutyric acid; 2-hydroxy-5,5-dimethyl-2-oxo-1,3,2-dioxophosphorinane; 3,9-dihydroxy-2,4,8,10-tetroxo-3,9-diphosphaspiro[5.5] undecane-3,9-dioxide; aminotris(methylenephosphonic) acid; ethylenediaminotetra (methylenephosphonic) acid; hexamethylenediaminotetra(methylenephosphonic) acid; diethylenetriaminopenta(methylenephosphonic) acid; etc.

Specific examples of compounds comprised by formula (I) are given in the examples hereinbelow.

Salts of general formula (I) can be synthesized, e.g., by reacting, in the presence of a suitable solvent (such as water, methyl alcohol, ethyl alcohol, acetonitrile, etc.) and at temperatures of from 0°C to the boiling point of the solvent used, n mols of a derivative of the 2,4,6-triamino-1,3,5-triazine of general formula (II): 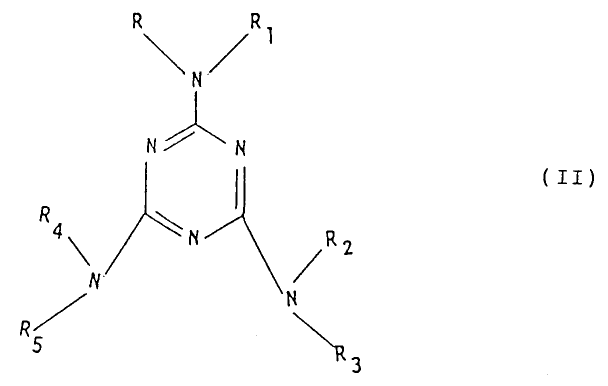
wherein n and the groups R and R₁ to R₅ have the previously defined meanings, with one mol of phosphorus-containing acid of general formula (III): 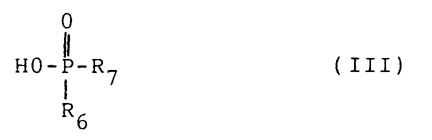
wherein R₆ and R₇ have the previously defined meanings, or without solvent and with an excess of the acid, when the latter can act as solvent, at temperatures of from about 0 to about 150°C.

The salt thus formed can easily be separated from the reaction mass, e.g., by filtration or by distilling off the solvent.

Generally products of general formula (I) of good quality are obtained in the form of a white crystalline powder. Said products are useable in self-extinguishing polymeric compositions without further purification.

Many of the intermediates of general formula (II) are known; however, they also can easily be synthesized according to the following general scheme: 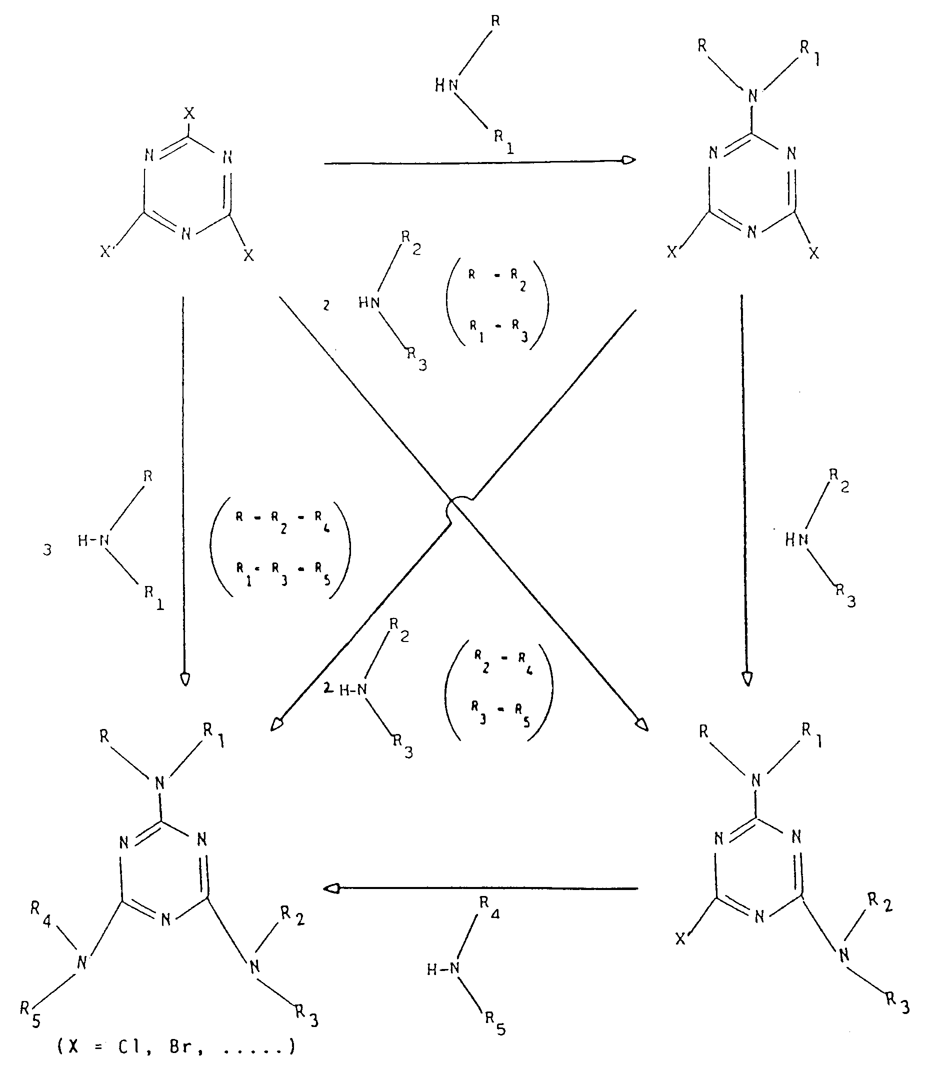
or according to the process disclosed in IT-A-21066 A/89.

The acids of general formula (III) are also known and many of them are also available in commercial quantities.

The following examples are to illustrate the present invention without limiting it.

The salification reactions between the intermediates of general formula (II) and the acids of general formula (III) were confirmed by IR-spectroscopic analyses carried out on a IR Perkin Elmer 580 B grid spectrophotometer.

In fact it has been observed that a very good reference signal is the band associated with the out of plane deformation of the triazine ring; this band is at about 830 to 800 cm⁻¹ for the undisturbed ring, while it is at 795 to 760 cm⁻¹ for the salified ring.

### EXAMPLE 1

Into a 3 l-reactor equipped with stirrer, thermometer, dropping funnel, reflux cooler and heating bath, there were introduced 184.5 g of cyanuric acid chloride and 800 ml of acetone.

The mixture was stirred while heating it to 40°C until a solution was obtained; thereafter, within 1.5 h and while keeping the temperature at 40°C, 284 g of aqueous ammonia (30% by weight) was added. The resulting mixture was then heated to 45°C and kept at this temperature for 4 hours.

After cooling, the formed product was filtered off and washed with water on the filter.

After drying in an oven at 50 to 60°C under vacuum, 115 g of the intermediate of formula (IV): 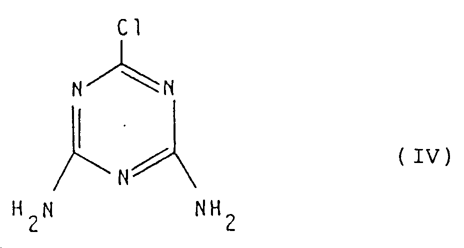
were obtained in the form of an infusible, white crystalline powder having a chlorine content of 24.12 % (theor. 24.36 %).

The structure of this product was further confirmed by IR spectroscopic analysis.

Into a 1 liter reactor equipped with stirrer, thermometer, dropping funnel, reflux cooler and heating bath, there were introduced 72.8 g of the intermediate (IV), 350 g of water and thereafter, under agitation, 44 g of piperidine.

The resulting mixture was heated to reflux and kept there for 4 hours.

Thereafter, still under reflux conditions, portions of 20 g of sodium hydroxide in 50 g of water were added over 8 hours in order to keep the pH between 7 and 8.

After cooling to room temperature, the formed product was filtered and washed on the filter with water.

After drying in an oven at 60°C under vacuum, 90 g of 2,4-diamino-6-piperidino-1,3,5-triazine were obtained in the form of a white crystalline powder, m.p. = 215 to 217°C (m.p. = melting point).

Into the same 1 liter reactor there were then introduced 77.6 g of the above 2,4-diamino-6-piperidino-1,3,5-triazine, 400 ml of water and, under stirring, 48.4 g of phosphoric acid (85 % by weight).

The resulting mixture was heated to 80°C and kept at this temperature for 4 hours.

After cooling to 10°C, the formed product was filtered and washed on the filter with water.

After drying in an oven at 100°C, 96 g of the product of formula 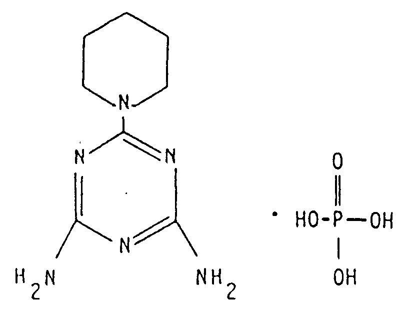
were obtained in the form of a crystalline powder having a m.p. of 228 to 230°C and a phosphorus content of 10.52 % (theor. 10.61 %).

### EXAMPLE 2

Into a 0.5 l-reactor, equipped like that described in example 1, there were introduced 49.0 g of the intermediate (IV), 150 ml of water and 26.2 g of 2-methoxyethylamine.

The mixture was heated to reflux and was kept there for 4 hours.

Thereafter, a solution of 14 g of sodium hydroxide in 50 ml of water was added within 20 minutes.

After stirring for a further 30 minutes, the distillation of water began; the residual mass was then treated with three 100 ml portions of acetonitrile in order to extract the organic product from the mass.

Upon subsequent distillation of the solvent, 52.5 g of 2,4-diamino-6-(2-methoxyethyl)amino-1,3,5-triazine were obtained as white powder (m.p. = 166 to 169°C).

Into the 1 l-reactor of example 1, there were introduced 52.5 g of 2,4-diamino-6-(2-methoxyethyl)amino-1,3,5-triazine, 600 ml of acetonitrile and, under stirring. 34.5 g of phosphoric acid (85% by weight).

The resulting mixture was heated to reflux and was kept there for 4 hours.

After cooling to room temperature, the formed product was filtered and washed on the filter with acetonitrile.

After drying in an oven at 100°C, 78 g of the product of formula 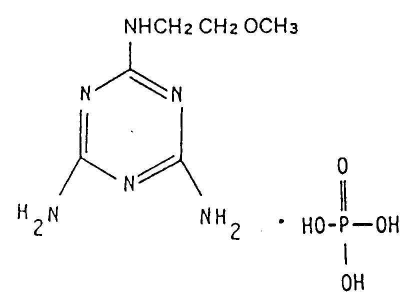
were obtained in the form of white crystalline powder having a m.p. of 186 to 188°C and a phosphorus content of 11.0% (theor. 10.98 %).

### EXAMPLE 3

Into the 1 l-reactor described in example 1 there were introduced 91 g of the intermediate (IV), 240 ml of toluene and 110 g of morpholine.

The mixture was heated to 65 to 70°C and kept at this temperature for 2 hours; thereafter, it was heated to reflux and was kept there for 1 hour.

Then the mixture was allowed to cool to room temperature and the formed product was separated by filtration. The cake was thoroughly washed with water and, after drying, 92 g of 2,4-diamino-6-morpholino-1,3,5-triazine were obtained in the form of a white crystalline powder having a m.p. of 248 to 250°C.

Into a 0.5 l-reactor equipped as described in example 1, there were introduced 39.9 g of the above 2,4-diamino-6-morpholino-1,3,5-triazine, 250 ml of acetonitrile and, under stirring, 24.2 g of phosphoric acid (85% by weight).

The resulting mixture was heated to reflux and was kept there for 8 hours.

Then, by working as described in example 2, 57 g of the product of formula 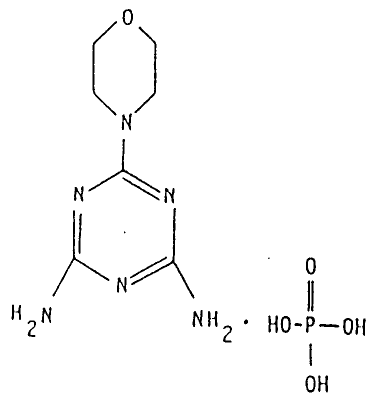
were obtained in the form of a white crystalline powder having a m.p. of 250 to 252°C and a phosphorus content of 10.5% (theor. 10.54 %).

### EXAMPLE 4

Into a 1 l-reactor equipped as described in example 1 there were introduced 72.8 g of the intermediate (IV), 250 ml of water and, under stirring, 104 g of thiomorpholine.

The resulting mixture was heated to reflux and was kept there for 8 hours.

Then the reaction mixture was cooled to room temperature and the formed product was filtered and the filter cake was washed with water.

After drying in an oven at 100°C, 90.2 g of 2,4-diamino-6-thiomorpholino-1,3,5-triazine were obtained in the form of a white crystalline powder; m.p. = 237 to 239°C.

Into the same 1 l-reactor there were introduced 41.4 g of the above 2,4-diamino-6-thiomorpholino-1,3,5-triazine, 300 ml of water and, under stirring, 32.5 g of phenylphosphonic acid.

The mixture was heated to 80°C and was kept at this temperature for 6 hours.

After cooling to room temperature, the formed product was filtered and washed on the filter with a small amount of water.

After drying in an oven at 100°C, 64.7 g of the product of formula 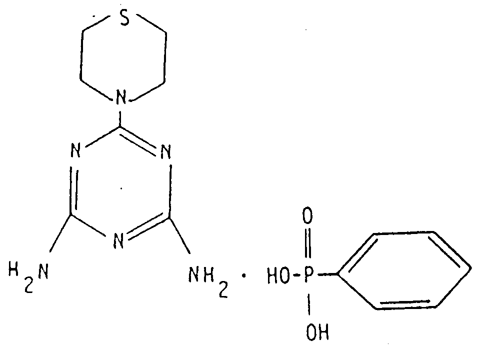
were obtained in the form of a white powder; m.p. 265 to 269°C; phosphorus content = 8.14 % (theor. 8.38 %).

### EXAMPLE 5

Into the 3 l-reactor of example 1, there were introduced 136 g of the intermediate (IV) and 800 ml of xylene.

The resulting suspension was heated to 120°C and within 1 h 302 g of the ethyl ester of N-piperazine carboxylic acid were added.

The reation mixture was kept at 125 to 130°C for 2 hours, then was cooled to room temperature and the formed product was filtered, washing the filter cake first with xylene and then thoroughly with water.

After drying in an oven at 100°C, 230 g of the intermediate of formula (V): 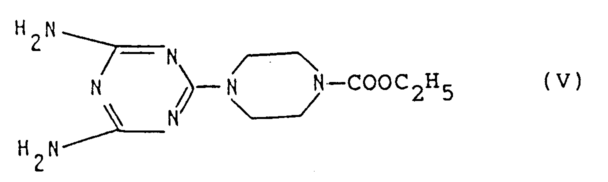
were obtained in the form of a white crystalline powder; m.p. = 210 to 215°C.

The structure of this intermediate was confirmed by NMR analysis.

Into the above 3 l-reactor there were introduced 1000 ml of acetic acid, 620 g of acetic solution of hydrobromic acid (33% by weight) and 120 g of the intermediate (V).

The resulting mixture was heated to 95°C and was kept at this temperature for 6 hours under stirring.

Thereafter the reaction mixture was cooled to room temperature and the formed product was filtered and washed on the filter with acetic acid.

The squeezed filter cake was then treated in a 2 l-beaker with 500 ml of water and, under stirring, a 50% by weight sodium hydroxide solution was added thereto until the pH reached 11.

The resulting mixture was stirred for a further hour and then the formed product was filtered and washed thoroughly on the filter with water.

After drying in an oven at 100°C, 60 g of 2,4-diamino-6-piperazino-1,3,5-triazine were obtained in the form of a white powder; m.p. = 262 to 268°C.

Into the 1 l-reactor of example 1, now equipped with a cooling bath, there were introduced 106.4 g of tetrasodium pyrophosphate and 600 ml of water.

The mixture was externally cooled to 5°C and thereafter 158 g of hydrochloric acid (37% by weight) were added, whereby a solution was obtained.

To this solution, always at 5°C, there were added 78 g of the above 2,4-diamino-6-piperazino-1,3,5-triazine.

The resulting mixture was stirred for 2 hours at 5°C whereafter it was heated to 10°C and was kept at this new temperature for 3 hours.

After cooling to 2°C the formed product was separated by filtration and the cake was washed on the filter with a small amount of cold water.

After drying in an oven at 100°C, 102 g of the product of formula 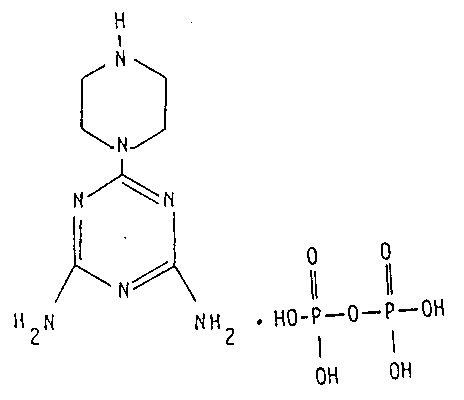
were obtained in the form of a white powder; m.p. = 295 to 298°C; phosphorus content equal to 16.8% (theor. 16.61 %).

### EXAMPLE 6

Into the 3 l-reactor described in example 1, but at the beginning equipped with a cooling bath, there were introduced 184.5 g of cyanuric acid chloride and 1300 ml of methylene chloride.

While cooling from the outside, 87.2 g of morpholine and 40 g of sodium hydroxide, dissolved in 150 g of water, were simultaneously introduced into the reactor within 3 hours, while keeping the pH between 5 to 7 and the temperature between 0 and 3°C.

The resulting mixture was further kept at a temperature of from 0 to 3°C for 3 hours and then the aqueous phase was separated.

By distillation of the methylene chloride, 230 g of the intermediate of formula (VI): 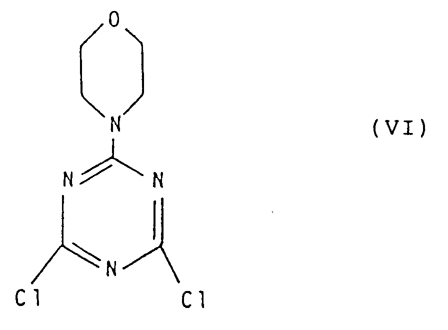
were obtained in the form of a white crystalline powder; m.p.= 155 to 157°C; titre higher than 98% (determined by gas chromatography); chlorine content equal to 29.87 % (theor. 30.21 %).

Into a 0.5 l-reactor, equipped like that described in example 1, there were introduced 100 g of aqueous ammonia (30% by weight), 100 ml of water and 70.5 g of the intermediate (VI).

The resulting mixture was heated to 50°C and was kept at this temperature for 7 hours; after having allowed the mixture to cool to room temperature, the obtained product was filtered and washed with water.

Upon drying the cake, 58 g of the intermediate of formula (VII): 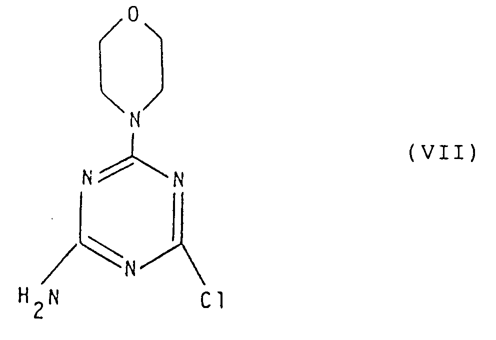
were obtained in the form of a white crystalline powder; m.p. = 189 to 191°C; chlorine content 16.28 % (theor. 16.47 %).

The structure of compounds (VI) and (VII) was confirmed by IR spectroscopic analysis.

Into the reactor described above there were introduced 58 g of the intermediate (VII) and 300 g of water and thereafter, under stirring, 18 g of 2-aminoethanol.

The resulting mixture was heated to reflux and was kept there for 3 hours.

Thereafter the mixture was further kept under reflux for 3 hours while adding in portions 11.8 g of sodium hydroxide dissolved in 50 g of water in order to maintain the pH between 7 and 8.

Then the reaction mixture was cooled, the obtained product was filtered and the filter cake was washed with water.

Upon drying 58 g of 2-amino-4-(2-hydroxyethyl)amino-6-morpholino-1,3,5-triazine, in the form of white powder, were obtained; m.p. = 159 to 161°C.

Into a 1 l-reactor,equipped like that of example 1, there were introduced 328 g of phosphorous acid and 82 g of acetonitrile and the reaction mixture was gradually heated, within 6 hours, up to 160°C, resulting in the formation of a white crystalline product.

Subsequently, the mixture was cooled to 80°C and 500 ml of water were added under vigorous stirring, followed by cooling to room temperature.

The formed product was separated by filtration and washed on the filter with a small amount of water.

Upon drying of the filter cake, 290 g of 1-aminoethane-1,1-diphosphonic acid were obtained in the form of a white crystalline powder; m.p. = 265 to 270°C (with decomposition); phosphorus content equal to 29.4% (theor. 30.24 %).

Into a 0.5 l-reactor, equipped like that described in example 1, there were introduced 200 ml of water, 36 g of 2-amino-4-(2-hydroxyethyl)amino-6-morpholino-1,3,5-triazine and 16 g of the above 1-aminoethane-1,1-diphosphonic acid.

The resulting mixture was heated to 80°C and was kept at this temperature for 1 hour, whereafter the distillation of the solvent was commenced.

Upon drying the solid remaining after said distillation in an oven at 100°C, there were obtained 51.6 g of the product of formula 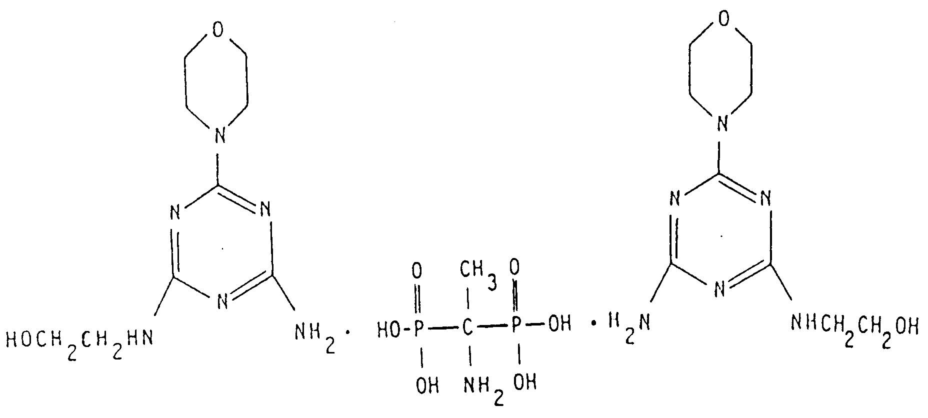
in the form of a white crystalline powder; m.p. = 144 to 148°C; phosphorus content 8.8% (theor. 9.04 %).

### EXAMPLE 7

Into a 2 l-reactor, equipped as described in the preceding examples, there were introduced 184.4 g of cyanuric acid chloride and 800 ml of methylene chloride.

By external cooling, the solution was kept at 4-5°C and within 2 hours 174 g of morpholine, dissolved in 150 ml of water, were added thereto.

The temperature was allowed to rise to 10°C and, while keeping it between 10 and 20°C, a solution of 80 g of sodium hydroxide in 200 g of water was added within 4 hours. The resulting mixture was kept at 20°C for a further 2 hours and thereafter the aqueous phase was removed.

By distilling the methylene chloride, 270 g of the intermediate of formula (VIII): 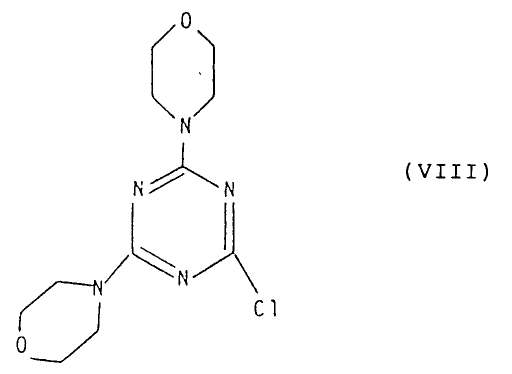
were obtained in the form of a white crystalline powder; m.p. = 172 to 174°C; chlorine content equal to 12.31 % (theor. 12.43 %).

The structure of this intermediate was confirmed by NMR analysis.

Into a 1 l-reactor , equipped as described in example 1, there were introduced 500 ml of water, 57.1 g of the intermediate (VIII) and, under stirring, 15.0 g of 2-methoxyethylamine.

The resulting mixture was heated to reflux and was kept there for 2 hours; thereafter, within about 1 hour, 8.0 g of sodium hydroxide, dissolved in 80 ml of water, were added.

After reflux for a further 4 hours, the reaction mixture was cooled to room temperature and the formed product was extracted with two 200 ml portions of methylene chloride.

By subsequent distillation of the solvent and drying of the distillation residue in an oven, 59.2 g of 2,4-dimorpholino-6-(2-methoxyethyl)amino-1,3,5-triazine were obtained; m.p. = 120 to 122°C.

Into a 0.5 l-reactor, equipped as described in example 1, there were introduced 48.6 g of the above 2,4-dimorpholino-6-(2-methoxyethyl)amino-1,3,5-triazine, 300 ml of acetonitrile and, under stirring, 17.4 g of phosphoric acid (85% by weight).

The resulting mixture was heated to reflux and was kept there for 4 hours.

Then the reaction mixture was cooled to room temperature and the formed product was filtered and washed on the filter with a small amount of acetonitrile.

Upon drying of the filter cake in an oven at 100°C, 63 g of the product of formula 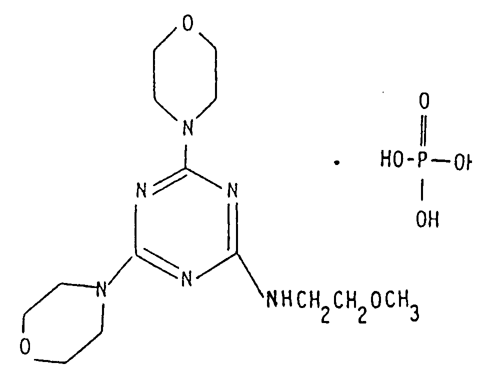
were obtained in the form of a white crystalline powder; m.p. = 179 to 182°C; phosphorus content equal to 7.46 % (theor. 7.33 %).

### EXAMPLE 8

Into a 0.5 l-reactor, equipped as described in example 1, there were introduced 39.2 g of 2,4-diamino-6-morpholino-1,3,5-triazine (prepared as described in example 3), 300 ml of ethanol and, under stirring, 17.2 g of phosphoric acid.

The resulting mixture was kept at room temperature for 8 hours under stirring; thereafter, the formed product was filtered and washed on the filter with a small amount of solvent.

Upon drying the filter cake in an oven at 100°C, 55 g of the product of formula 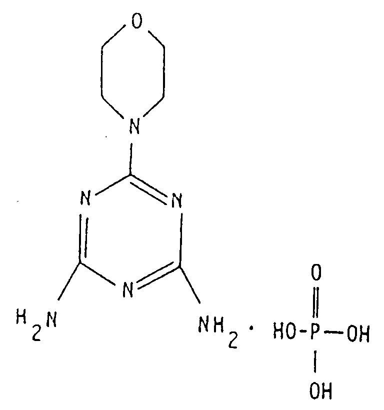
were obtained in the form of a white crystalline powder; m.p. = 240 to 244°C; phosphorus content equal to 11.0% (theor. 11.13 %).

### EXAMPLES 9 to 36

By working under conditions analogous to those described in examples 1 to 8, the salts of general formula (I) listed in Table 1 were prepared.

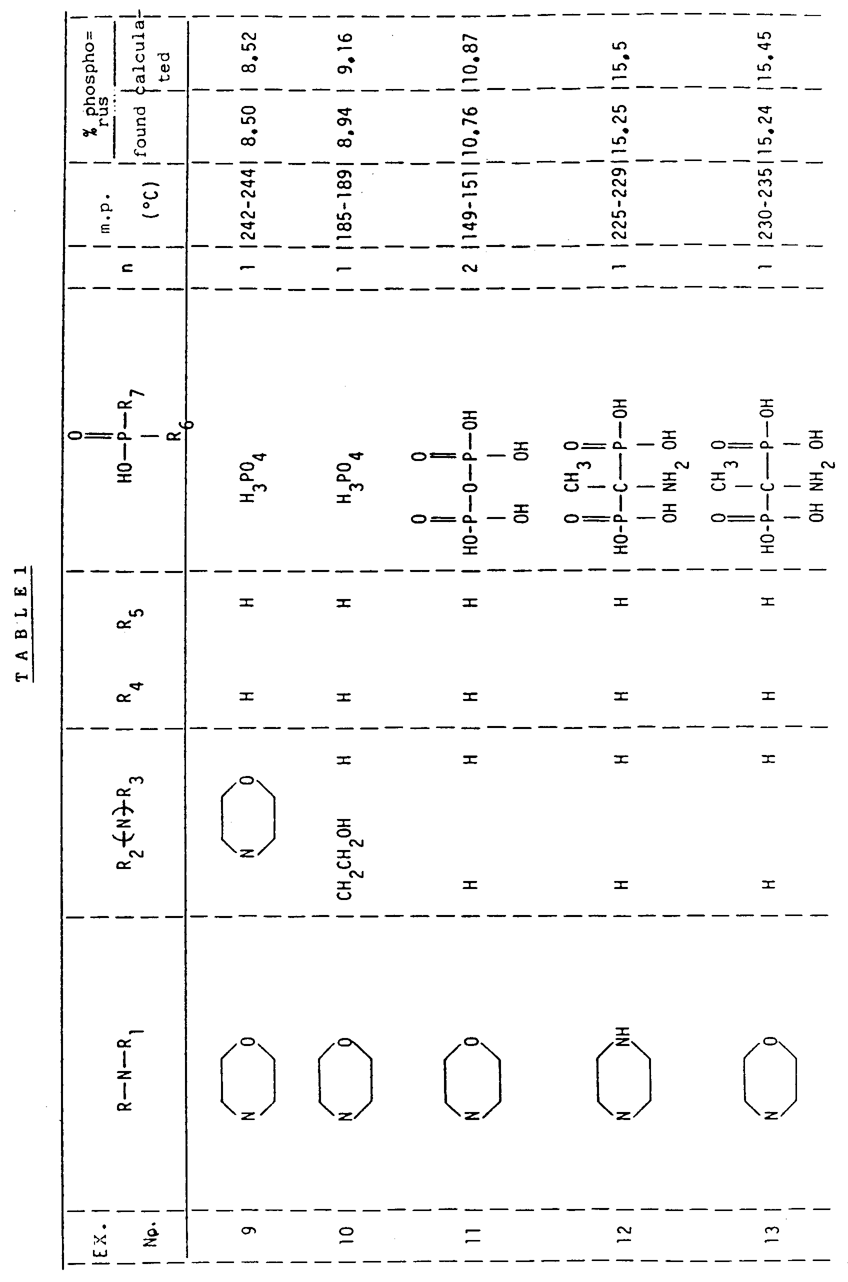

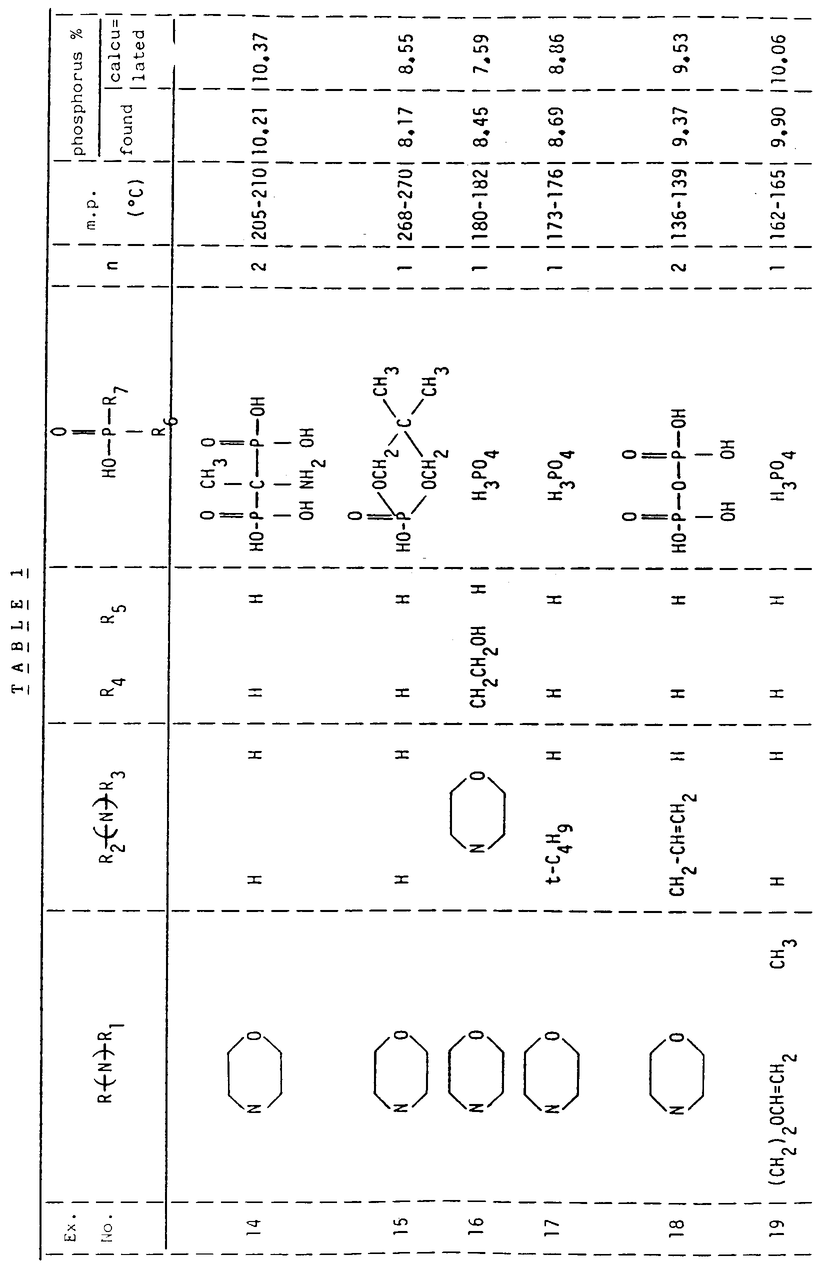

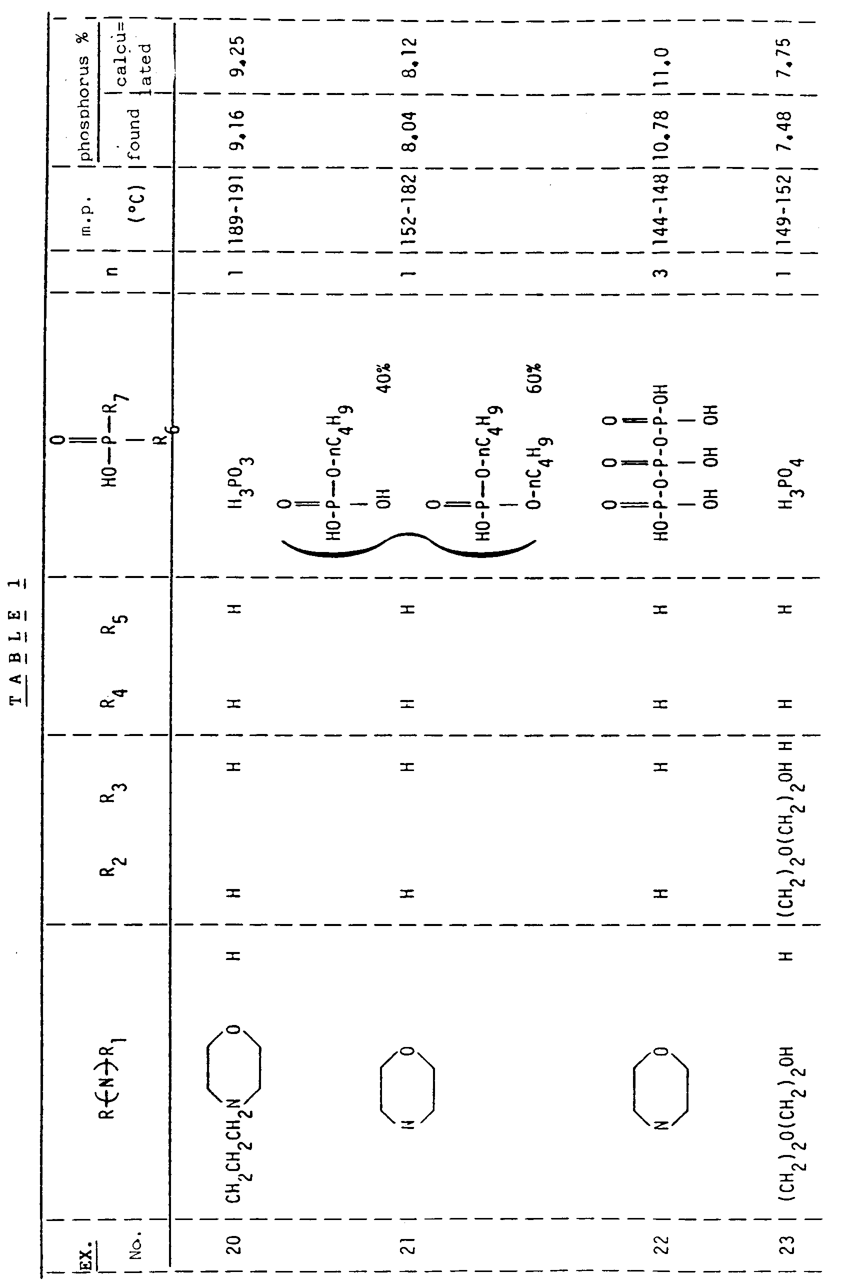

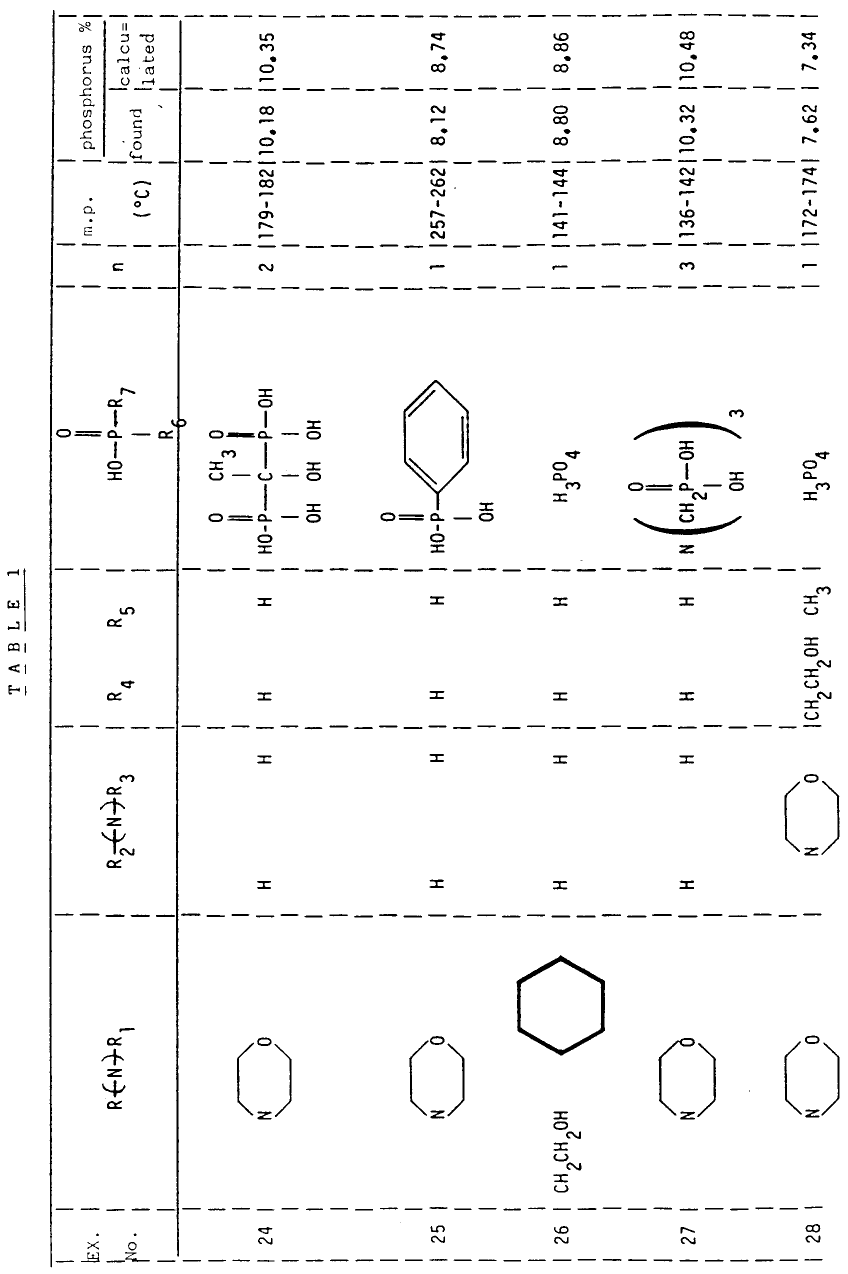

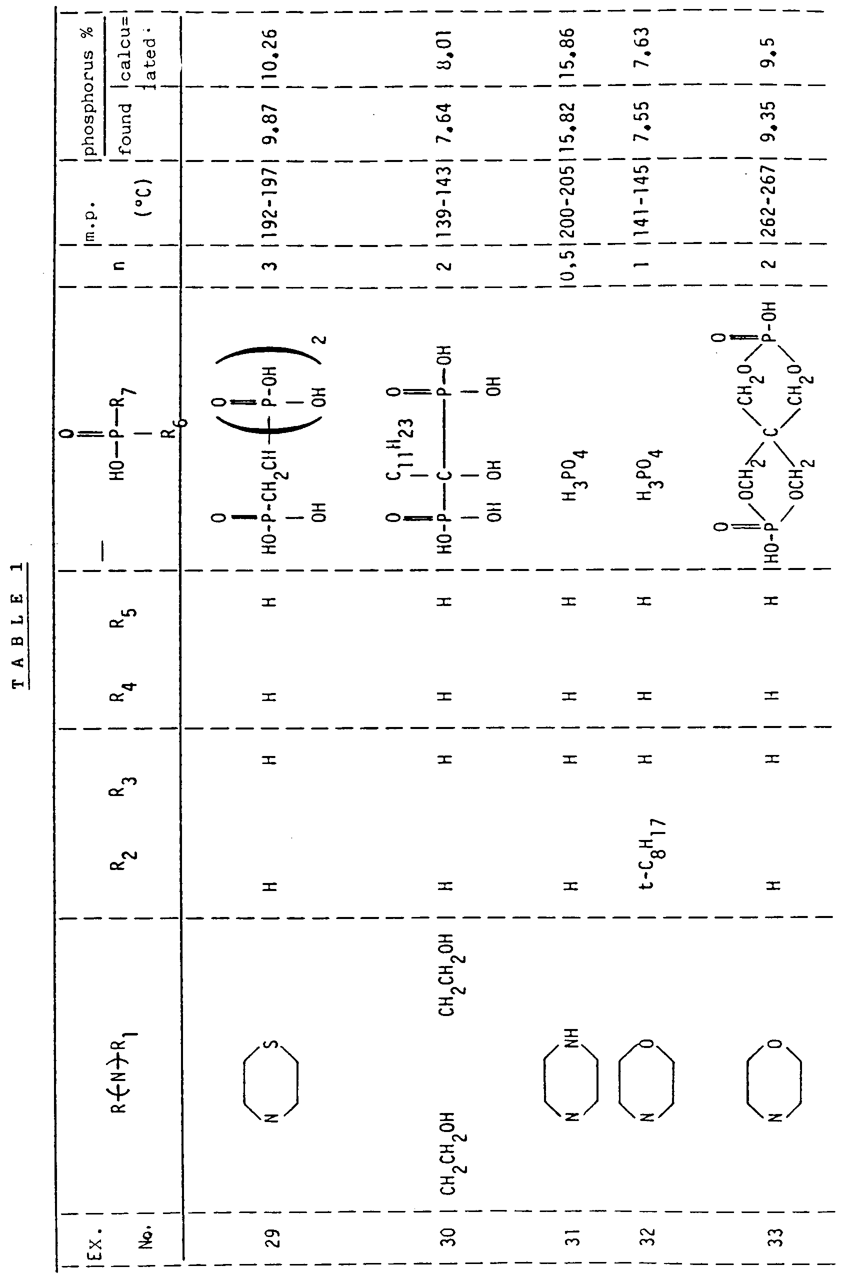

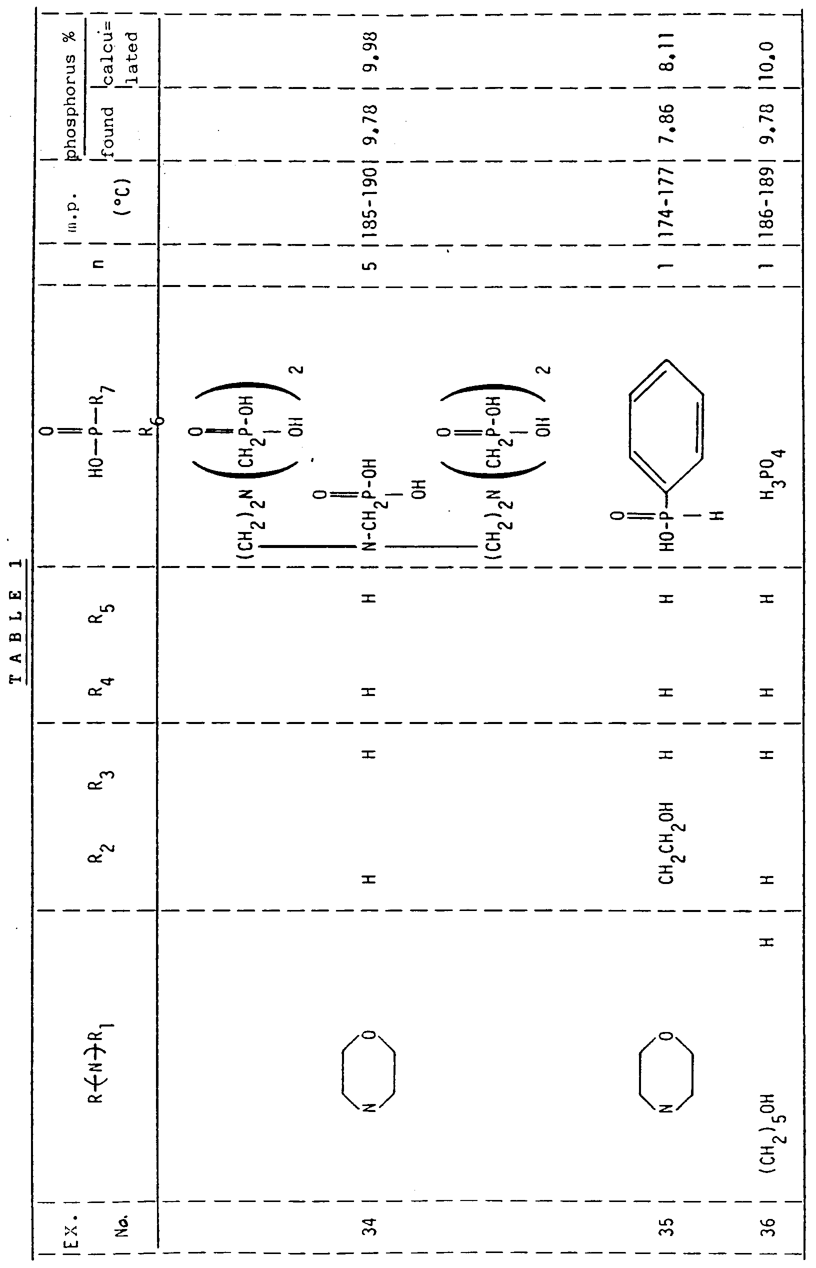

### EXAMPLE 37

60 g of isotactic polypropylene flakes (having a melt flow index of 12 and an insoluble fraction in boiling n-heptane of 96% by weight), 39 g of the product of example 3, 0.67 g of dilaurylthiopropionate and 0.33 g of pentaerythritol tetra [3-(3,5-di-tert.-butyl-4-hydroxyphenyl)propionate] were mixed and the resulting blend was molded in a MOORE platen press, by working for 7 minutes under a pressure of 40 kg/cm².

Samples in form of plates having a thickness of about 3 mm were obtained, on which the self-extinguishing level was determined by measuring the oxygen index (L.O.I. according to the ASTM D-2863/77) in a STANTON REDCROFT apparatus, and by applying the "Vertical Burning Test" which allows to classify the material according to three ratings, i.e., V-0, V-1 and V-2 (compare standard UL 94 published by Underwriter Laboratories - USA).

The following results were obtained:
L.O.I. = 35.4
UL 94 : Class V-0

## Claims (Claims for the following Contracting State(s): AT, BE, CH, DE, DK, FR, GB, LI, NL, SE)

1. Salts of triazine derivatives with oxygenated acids of phosphorus, having general formula (I): 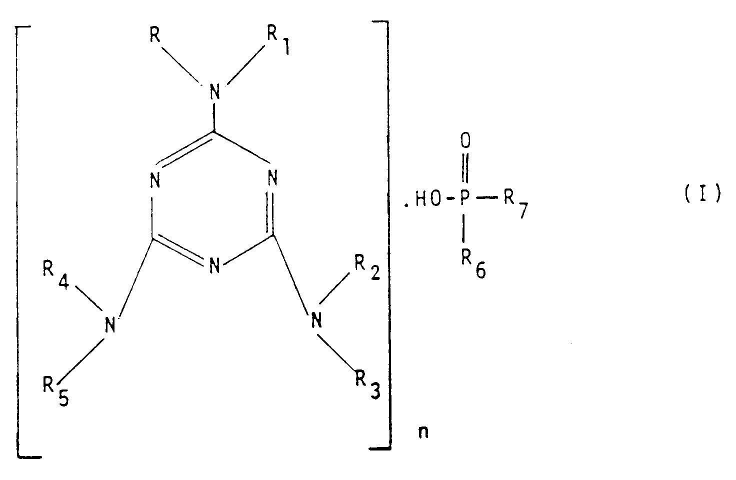 wherein at least one of the groups R and R₁ to R₅ is selected from
⁅CₘH₂ₘ⁆O-R₈ and 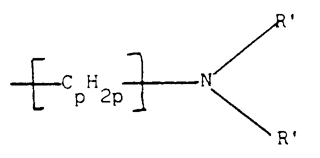 wherein
m = integer of from 2 to 8;
p = integer of from 2 to 6;
R₈ = H; (C₁-C₈) alkyl; (C₂-C₆) alkenyl; ⁅C_{q}H_{2q}⁆O-R₉,
q being an integer of from 1 to 4 and
R₉ being hydrogen or (C₁-C₄) alkyl; (C₆-C₁₂) cycloalkyl or (C₆-C₁₂) alkylcycloalkyl;
the groups R', the same or different from each other, are selected from H; (C₁-C₈) alkyl; (C₂-C₆) alkenyl;
(C₆-C₁₂) cycloalkyl or (C₆-C₁₂) alkylcycloalkyl; and
(C₁-C₄) hydroxyalkyl, provided that the groups R₆ and R₇, specified hereinafter, are different from H and OH, respectively;
or the moiety N(R')₂ is replaced by an N-heterocyclic radical which optionally contains another heteroatom and is bound to the alkyl chain through the nitrogen atom;
or in general formula (I) at least one of the groups NRR₁, NR₂R₃ and NR₄R₅ is replaced by an N-heterocyclic radical which optionally contains another heteroatom and is bound to the triazine ring through the nitrogen atom;
the remaining groups R and R₁ to R₅, the same or different from each other, have the meanings given above or are selected from H; (C₁-C₁₈) alkyl; (C₂-C₈) alkenyl;
(C₆-C₁₆) cycloalkyl or (C₆-C₁₆) alkylcycloalkyl, optionally substituted by a hydroxy or (C₁-C₄) hydroxyalkyl group;
n is a number from 0.5 to 6;
R₆ is selected from H; OH; (C₁-C₈) alkoxy; aryloxy, optionally substituted by a (C₁-C₈) alkyl group; aralkyl, optionally substituted by a (C₁-C₄) alkyl group; (C₁-C₄) alkyl, optionally substituted by a carboxylic group; and aryl;
R₇ is selected from H; OH; (C₁-C₈) alkoxy; aryloxy; (C₁-C₄) alkyl; aryl; a group of formula 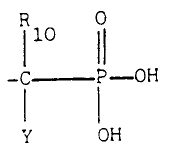
wherein R₁₀ is hydrogen or (C₁-C₁₂) alkyl and Y is OH or R₁₀;
a group of formula 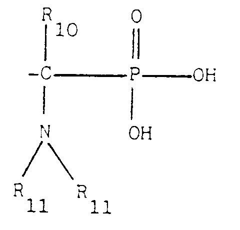 wherein R₁₀ is as defined above and the groups R₁₁, the same or different from each other, represent hydrogen or (C₁-C₄) alkyl or the moiety N(R₁₁)₂ is replaced by an N-heterocyclic radical which optionally contains another heteroatom and is bound to the carbon atom through the nitrogen atom;
a group of formula 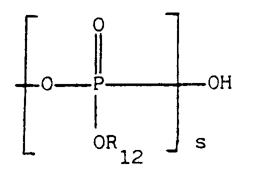 wherein R₁₂ is hydrogen or (C₁-C₈) alkyl and s is an integer of from 1 to 3;
a group of formula 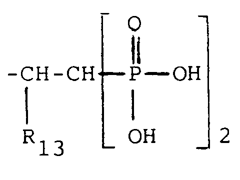 wherein R₁₃ is hydrogen or hydroxy;
a group of formula 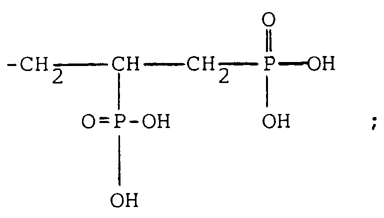 a group of formula 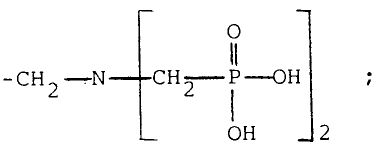 a group of formula 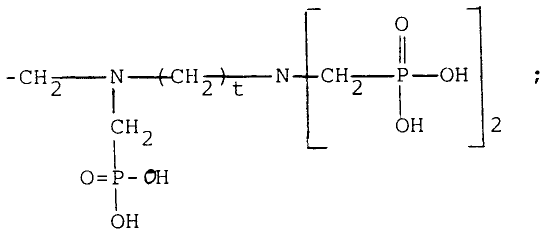 and a group of formula 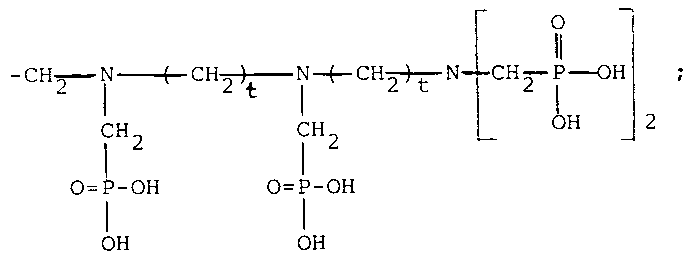 wherein t is an integer of from 2 to 6;
or R₆ and R₇ together form a cyclic structure of one of the following formulae:

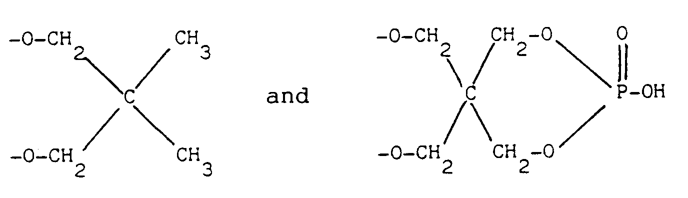

2. Salts according to claim 1, wherein one or more of the moieties NRR₁, NR₂R₃ and NR₄R₅ in general formula (I) represent heterocyclic radicals selected from aziridinyl; pyrrolidinyl, piperidinyl, morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl; 2- ethylpiperazinyl; and 2,5-diethylpiperazinyl.

3. Salts according to anyone of claims 1 and 2, wherein the moiety N(R')₂ is a heterocyclic radical selected from aziridinyl; pyrrolidinyl; piperidinyl; morpholinyl; thiomorpholinyl; piperazinyl; 4-methylpiperazinyl; and 4-ethylpiperazinyl.

4. Salts according to anyone of claims 1 to 3, wherein the acid of phosphorus is selected from
hypophosphorous acid; phosphorous acid; phosphoric acid; pyrophosphoric acid; tripolyphosphoric acid; ethane-1,1,2-triphosphonic acid; 2-hydroxyethane-1,1,2-triphosphonic acid; propane-1,2,3-triphosphonic acid; isopropylphosphoric acid; n-butylphosphoric acid; n-butylphosphoric acid; di-n-butylphosphoric acid; di-isopropylphosphoric acid; di-n-pentylphosphoric acid; isooctylphosphoric acid; ethylphosphoric acid; hexylphosphoric acid; 2-ethylhexylphosphoric acid; methylphosphonic acid; ethylphosphonic acid; n-propylphosphonic acid; n-butylphosphonic acid; aminomethylphosphonic acid; phenylphosphoric acid; phenylphosphonic acid; phenylphosphinic acid; di-n-butylpyrophosphoric acid; di(2-ethylhexyl)pyrophosphoric acid; octylphenylphosphoric acid; 2-methylbenzylphosphonic acid; 1-aminoethane-1,1-diphosphonic acid; 1-hydroxyethane-1,1-diphosphonic acid; 1-hydroxydodecane-1,1-diphosphonic acid; 1-(N-methylamino)ethane-1,1-diphosphonic acid; N,N-dimethylaminomethane-1,1-diphosphonic acid; N-butylaminomethane-1,1-diphosphonic acid; phosphonoacetic acid; 2-phosphonopropionic acid; 3-phosphonopropionic acid; 2-phosphonobutyric acid; 4-phosphonobutyric acid; 2-hydroxy-5,5-dimethyl-2-oxo-1,3,2-dioxophosphorinane; 3,9-dihydroxy-2,4,8,10-tetroxo-3,9-diphosphaspiro[5.5] undecane-3,9-dioxide; amino-tris(methylenephosphonic) acid; ethylenediaminotetra(methylenephosphonic) acid; hexamethylenediaminotetra (methylenephosphonic) acid; and diethylenetriaminopenta (methylenephosphonic) acid.

5. Process for the preparation of salts of general formula (I) according to anyone of claims 1 to 4, comprising the reaction of n mols of a derivative of 2,4,6-triamino-1,3,5-triazine of general formula (II): 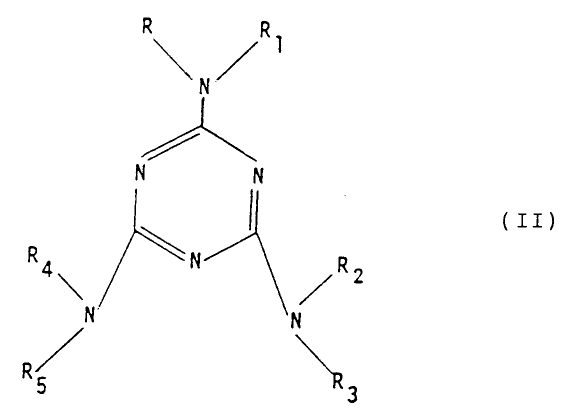 wherein n and the substituents R and R₁ to R₅ have the meanings given in claim 1, with 1 mol of a phosphorus -containing acid of general formula (III): 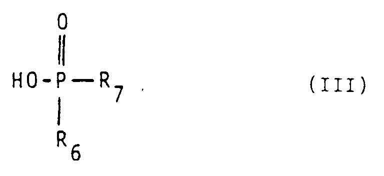 wherein R₆ and R₇ have the meanings given in claim 1.

6. Process according to claim 5, wherein the reaction between the derivatives of general formula (II) and the acids of general formula (III) is carried out in the presence of a solvent and at temperatures ranging from O°C to the boiling point of the solvent used.

## Claims (Claims for the following Contracting State(s): ES)

1. Process for the preparation of salts of triazine derivatives with oxygenated acids of phosphorus, having general formula (I): 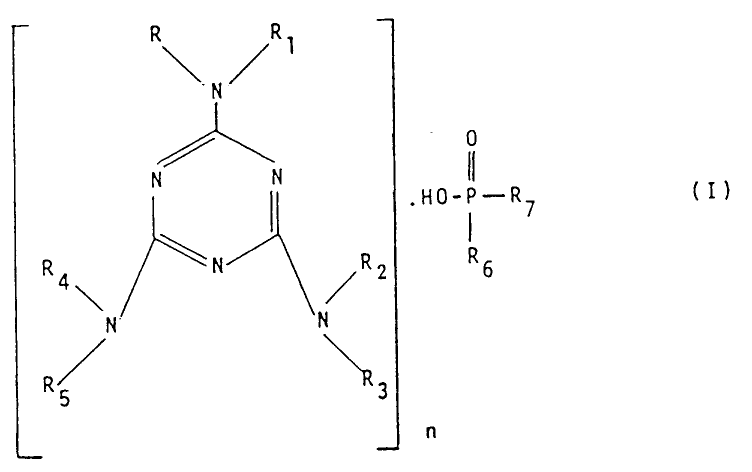 wherein at least one of the groups R and R₁ to R₅ is selected from
⁅CₘH₂ₘ⁆O-R₈ and 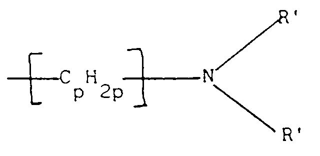 wherein
m = integer of from 2 to 8;
p = integer of from 2 to 6;
R₈ = H; (C₁-C₈) alkyl; (C₂-C₆) alkenyl; ⁅C_{q}H_{2q}⁆O-R₉,
q being an integer of from 1 to 4 and
R₉ being hydrogen or (C₁-C₄) alkyl; (C₆-C₁₂) cycloal]yl or (C₆-C₁₂) alkylcycloalkyl;
the groups R', the same or different from each other, are selected from H; (C₁-C₈) alkyl; (C₂-C₆) alkenyl; (C₆-C₁₂) cycloalkyl or (C₆-C₁₂) alkylcycloalkyl; and
(C₁-C₄) hydroxyalkyl, provided that the groups R₆ and R₇, specified hereinafter, are different from H and OH, respectively;
or the moiety N(R')₂ is replaced by an N-heterocyclic radical which optionally contains another heteroatom and is bound to the alkyl chain through the nitrogen atom;
or in general formula (I) at least one of the groups NRR₁, NR₂R₃ and NR₄R₅ is replaced by an N-heterocyclic radical which optionally contains another heteroatom and is bound to the triazine ring through the nitrogen atom;
the remaining groups R and R₁ to R₅, the same or different from each other, have the meanings given above or are selected from H; (C₁-C₁₈) alkyl; (C₂-C₈) alkenyl;
(C₆-C₁₆) cycloalkyl or (C₆-C₁₆) alkylcycloalkyl, optionally substituted by a hydroxy or (C₁-C₄) hydroxyalkyl group;
n is a number from 0.5 to 6;
R₆ is selected from H; OH; (C₁-C₈) alkoxy; aryloxy, optionally substituted by a (C₁-C₈) alkyl group; aralkyl, optionally substituted by a (C₁-C₄) alkyl group; (C₁-C₄) alkyl, optionally substituted by a carboxylic group; and aryl;
R₇ is selected from H; OH; (C₁-C₈) alkoxy; aryloxy; (C₁-C₄) alkyl; aryl; a group of formula 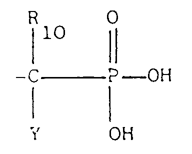
wherein R₁₀ is hydrogen or (C₁-C₁₂) alkyl and Y is OH or R₁₀;
a group of formula 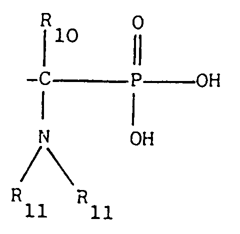 wherein R₁₀ is as defined above and the groups R₁₁, the same or different from each other, represent hydrogen or (C₁-C₄) alkyl or the moiety N(R₁₁)₂ is replaced by an N-heterocyclic radical which optionally contains another heteroatom and is bound to the carbon atom through the nitrogen atom;
a group of formula 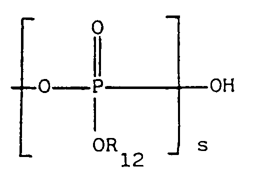 wherein R₁₂ is hydrogen or (C₁-C₈) alkyl and s is an integer of from 1 to 3;
a group of formula 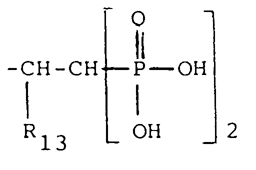 wherein R₁₃ is hydrogen or hydroxy;
a group of formula 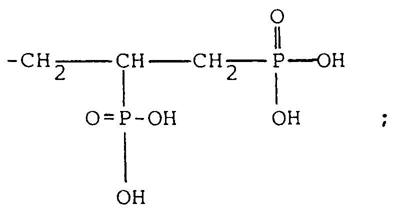 a group of formula 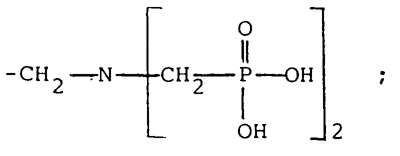 a group of formula 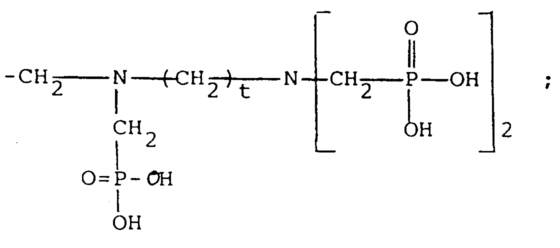 and a group of formula 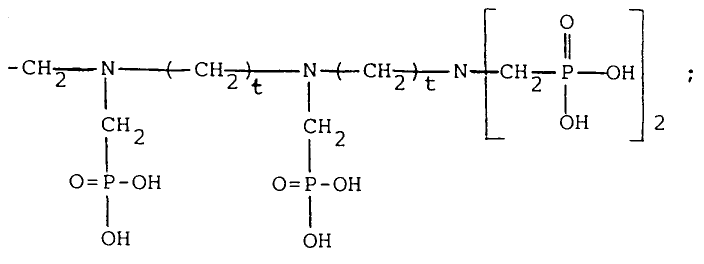 wherein t is an integer of from 2 to 6;
or R₆ and R₇ together form a cyclic structure of one of the following formulae: 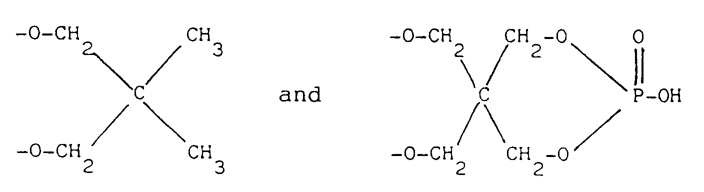 comprising the reaction of n mols of a derivative of 2,4,6-triamino-1,3,5-triazine of general formula (II): 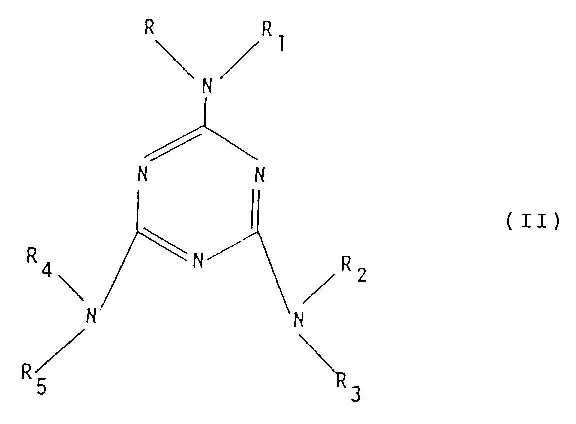 wherein n and the substituents R and R₁ to R₅ have the meanings given hereinbefore, with 1 mol of a phosphorus-containing acid of general formula (III): 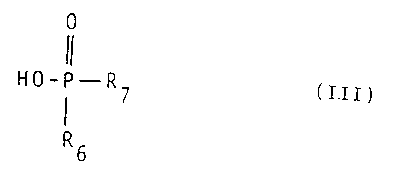 wherein R₆ and R₇ have the meaning given hereinbefore.

2. Process according to claim 1, wherein one or more of the moieties NRR₁, NR₂R₃ and NR₄R₅ in general formula (I) represent heterocyclic radicals selected from aziridinyl; pyrrolidinyl, piperidinyl, morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl; 2- ethylpiperazinyl; and 2,5-diethylpiperazinyl.

3. Process according to anyone of claims 1 and 2, wherein the moiety N(R')₂ is a heterocyclic radical selected from aziridinyl; pyrrolidinyl; piperidinyl; morpholinyl; thiomorpholinyl; piperazinyl; 4-methylpiperazinyl; and 4-ethylpiperazinyl.

4. Process according to anyone of claims 1 to 3, wherein the acid of phosphorus is selected from
hypophosphorous acid; phosphorous acid; phosphoric acid; pyrophosphoric acid; tripolyphosphoric acid; ethane-1,1,2-triphosphonic acid; 2-hydroxyethane-1,1,2-triphosphonic acid; propane-1,2,3-triphosphonic acid; isopropylphosphoric acid; n-butylphosphoric acid; n-butylphosphoric acid; di-n-butylphosphoric acid; di-isopropylphosphoric acid; di-n-pentylphosphoric acid; isooctylphosphoric acid; ethylphosphoric acid; hexylphosphoric acid; 2-ethylhexylphosphoric acid; methylphosphonic acid; ethylphosphonic acid; n-propylphosphonic acid; n-butylphosphonic acid; aminomethylphosphonic acid; phenylphosphoric acid; phenylphosphonic acid; phenylphosphinic acid; di-n-butylpyrophosphoric acid; di(2-ethylhexyl)pyrophosphoric acid; octylphenylphosphoric acid; 2-methylbenzylphosphonic acid; 1-aminoethane-1,1-diphosphonic acid; 1-hydroxyethane-1,1-diphosphonic acid; 1-hydroxydodecane-1,1-diphosphonic acid; 1-(N-methylamino)ethane-1,1-diphosphonic acid; N,N-dimethylaminoethane-1,1-diphosphonic acid; N-butylaminomethane-1,1-diphosphonic acid; phosphonoacetic acid; 2-phosphonopropionic acid; 3-phosphonopropionic acid; 2-phosphonobutyric acid; 4-phosphonobutyric acid; 2-hydroxy-5,5-dimethyl-2-oxo-1,3,2-dioxophosphorinane; 3,9-dihydroxy-2,4,8,10-tetroxo-3,9-diphosphaspiro[5.5] undecane-3,9-dioxide; amino-tris(methylenephosphonic) acid; ethylenediaminotetra(methylenephosphonic) acid; hexamethylenediaminotetra (methylenephosphonic) acid; and diethylenetriaminopenta (methylenephosphonic) acid.

5. Process according to anyone of claims 1-4, wherein the reaction between the derivatives of general formula (II) and the acids of general formula (III) is carried out in the presence of a solvent and at temperatures ranging from 0°C to the boiling point of the solvent used.

6. Salts of triazine derivatives with oxygenated acids of phosphorus, having general formula (I) as defined in claim 1.

## Patentansprüche (Patentansprüche für folgende(n) Vertragsstaat(en): AT, BE, CH, DE, DK, FR, GB, LI, NL, SE)

1. Salze von Triazin-Derivaten mit sauerstoffhaltigen Säuren des Phosphors mit der allgemeinen Formel (I): 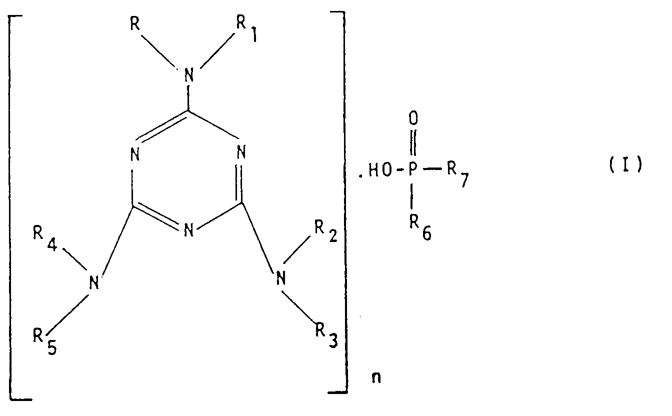 worin wenigstens eine der Gruppen R und R₁ bis R₅ ausgewählt ist aus -[-CₘH₂ₘ-]-O-R₈ und
-[-CₚH₂ₚ-]-N(R')₂
worin
m = eine ganze Zahl zwischen 2 und 8,
p = eine ganze Zahl zwischen 2 und 6,
R₈ = H, (C₁-C₈)-Alkyl, (C₂-C₆)-Alkenyl, -[-C_{q}H_{2q}-]-O-R₉, wobei
q eine ganze Zahl zwischen 1 und 4 und
R₉ Wasserstoff oder (C₁-C₄)-Alkyl, (C₆-C₁₂)-Cycloalkyl oder (C₆-C₁₂)-Alkylcycloalkyl bedeutet,
die Gruppen R', gleich oder voneinander verschieden, ausgewählt sind aus H, (C₁-C₈)-Alkyl, (C₂-C₆)-Alkenyl, (C₆-C₁₂)-Cycloalkyl oder (C₆-C₁₂)-Alkylcycloalkyl und (C₁-C₄)-Hydroxyalkyl, vorausgesetzt, daß die nachfolgend definierten Gruppen R₆ und R₇ ungleich H bzw. OH sind,
oder die Gruppe N(R')₂ durch einen Rest eines N-Heterocyclus ersetzt ist, der gegebenenfalls ein anderes Heteroatom enthält und an die Alkylkette durch das Stickstoffatom gebunden ist,
oder in der allgemeinen Formel (I) wenigstens eine der Gruppen NRR₁, NR₂R₃ und NR₄R₅ durch einen Rest eines N-Heterocyclus ersetzt ist, der gegebenenfalls ein anderes Heteroatom enthält und an den Triazinring durch das Stickstoffatom gebunden ist,
die restlichen Gruppen R und R₁ bis R₅, gleich oder voneinander verschieden, die oben angegebenen Bedeutungen haben oder ausgewählt sind aus H, (C₁-C₁₈)-Alkyl, (C₂-C₈)-Alkenyl, (C₆-C₁₆)-Cycloalkyl oder (C₆-C₁₆)-Alkylcycloalkyl, gegebenenfalls substituiert mit einer Hydroxy- oder (C₁-C₄)-Hydroxyalkylgruppe,
n eine Zahl zwischen 0.5 und 6 ist,
R₆ ausgewählt ist aus H, OH, (C₁-C₈)-Alkoxy, Aryloxy, gegebenenfalls substituiert durch eine (C₁-C₈)-Alkylgruppe, Aralkyl, gegebenenfalls substitiert mit einer (C₁-C₄)-Alkylgruppe, (C₁-C₄)-Alkyl, gegebenenfalls substituiert mit einer Carboxylgruppe, und Aryl,
R₇ ausgewählt ist aus H, OH, (C₁-C₈)-Alkoxy, Aryloxy, (C₁-C₄)-Alkyl, Aryl, eine Gruppe der Formel 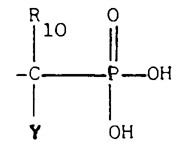
worin R₁₀ Wasserstoff oder (C₁-C₁₂)-Alkyl ist und Y OH oder R₁₀,
eine Gruppe der Formel 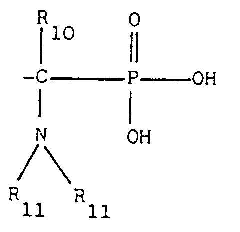 worin R₁₀ wie oben definiert ist und die Gruppen R₁₁, gleich oder verschieden voneinander, Wasserstoff oder (C₁-C₄)-Alkyl darstellen oder die Einheit N(R₁₁)₂ durch einen Rest eines N-Heterocyclus ersetzt ist, der gegebenenfalls ein anderes Heteroatom enthält und an das Kohlenstoffatom durch das Stickstoffatom gebunden ist,
eine Gruppe der Formel 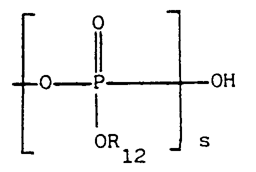 worin R₁₂ Wasserstoff oder (C₁-C₈)-Alkyl ist und s eine ganze Zahl von 1 bis 3,
eine Gruppe der Formel 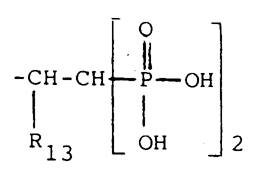 worin R₁₃ Wasserstoff oder Hydroxy ist,
eine Gruppe der Formel 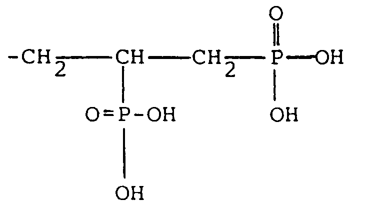 eine Gruppe der Formel 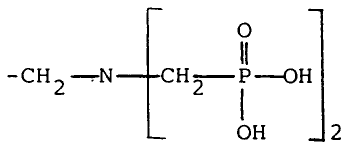 eine Gruppe der Formel 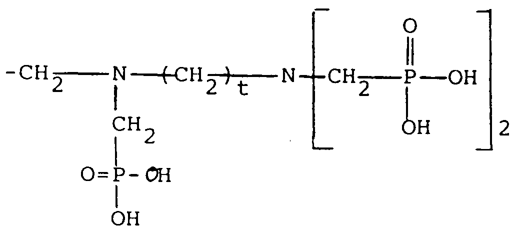 und eine Gruppe der Formel 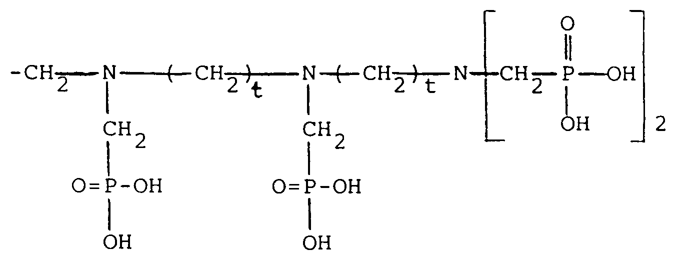 worin t eine ganze Zahl zwischen 2 und 6 ist;
oder R₆ und R₇ zusammen eine cyclische Struktur einer der folgenden Formeln bilden:

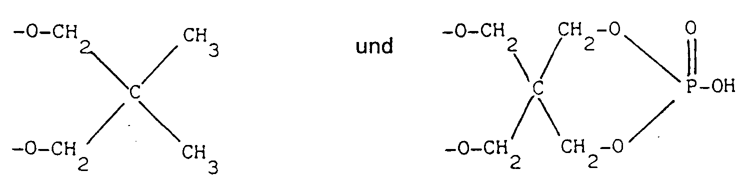

2. Salze gemäß Anspruch 1, worin eine oder mehrere der Einheiten NRR₁, NR₂R₃ und NR₄R₅ in der allgemeinen Formel (I) heterocyclische Reste darstellen, die ausgewählt sind aus Aziridinyl, Pyrrolidinyl, Piperidinyl, Morpholinyl, Thiomorpholinyl, Piperazinyl, 4-Methylpiperazinyl, 4-Ethylpiperazinyl, 2-Methylpiperazinyl, 2,5-Dimethylpiperazinyl, 2,3,5,6-Tetramethylpiperazinyl, 2,2,5,5-Tetramethylpiperazinyl, 2-Ethylpiperazinyl und 2,5-Diethylpiperazinyl.

3. Salze gemäß irgendeinem der Ansprüche 1 und 2, worin die Einheit N(R')₂ einen heterocyclischen Rest darstellt, der ausgewählt ist aus Aziridinyl, Pyrrolidinyl, Piperidinyl, Morpholinyl, Thiomorpholinyl, Piperazinyl, 4-Methylpiperazinyl und 4-Ethylpiperazinyl.

4. Salze gemäß irgendeinem der Ansprüche 1 bis 3, worin die Säure des Phosphors ausgewählt ist aus Hypophosphorsäure, Phosphoriger Säure, Phosphorsäure, Pyrophosphorsäure, Tripolyphosphorsäure, Ethan-1,1,2-triphosphonsäure, 2-Hydroxyethan-1,1,2-triphosphonsäure, Propan-1,2,3-triphosphonsäure, Isopropylphosphorsäure, n-Butylphosphorsäure, n-Butylphosphorsäure, Di-n-butylphosphorsäure, Diisopropylphosphorsäure, Di-n-pentylphosphorsäure, Isooctylphosphorsäure, Ethylphosphorsäure, Hexylphosphorsäure, 2-Ethylhexylphosphorsäure, Methylphosphonsäure, Ethylphosphonsäure, n-Propylphosphonsäure, n-Butylphosphonsäure, Aminomethylphosphonsäure, Phenylphosphorsäure, Phenylphosphonsäure, Phenylphosphinsäure, Di-n-butylpyrophosphorsäure, Di(2-ethylhexyl)pyrophosphorsäure, Octylphenylphosphorsäure, 2-Methylbenzylphosphonsäure, 1-Aminoethan-1,1-diphosphonsäure, 1-Hydroxyethan-1,1-diphosphonsäure, 1-Hydroxydodecan-1,1-diphosphonsäure,1-(N-Methylamino)ethan-1,1-diphosphonsäure,N,N-Dimethylaminomethan-1,1-diphosphonsäure, N-Butylaminomethan-1,1-diphosphonsäure, Phosphonoessigsäure, 2-Phosphonopropionsäure, 3-Phosphonopropionsäure, 2-Phosphonobuttersäure, 4-Phosphonobuttersäure, 2-Hydroxy-5,5-dimethyl-2-oxo-1,3,2-dioxophosphorinan, 3,9-Dihydroxy-2,4,8,10-tetroxo-3,9-diphosphaspiro[5.5]undecan-3,9-dioxid,Amino-tris(methylenphosphon)säure, Ethylendiaminotetra(methylenphosphon)säure, Hexamethylendiaminotetra(methylenphosphon)säure und Diethylentriaminopenta(methylenphosphon)säure.

5. Verfahren zur Herstellung der Salze der allgemeinen Formel (I) gemäß irgendeinem der Ansprüche 1 bis 4, umfassend die Umsetzung von n Mol eines Derivats von 2,4,6-Triamino-1,3,5-triazin der allgemeinen Formel (II): 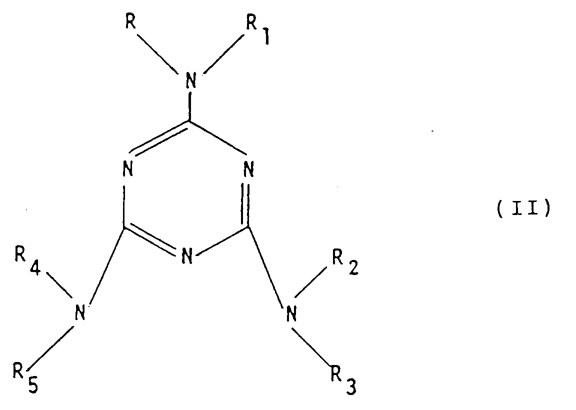 worin n und die Substituenten R und R₁ bis R₅ die in Anspruch 1 angegebenen Bedeutungen haben, mit 1 Mol einer phosphorhaltigen Säure der allgemeinen Formel (III): 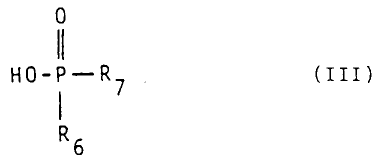 worin R₆ und R₇ die in Anspruch 1 genannten Bedeutungen besitzen.

6. Verfahren gemäß Anspruch 5, worin die Umsetzung zwischen den Derivaten der allgemeinen Formel (II) und den Säuren der allgemeinen Formel (III) in Gegenwart eines Lösemittels und bei einer Temperatur im Bereich von 0 °C bis zum Siedepunkt des verwendeten Lösemittels durchgeführt wird.

## Patentansprüche (Patentansprüche für folgende(n) Vertragsstaat(en): ES)

1. Verfahren zur Herstellung von Salzen von Triazin-Derivaten mit sauerstoffhaltigen Säuren des Phosphors mit der allgemeinen Formel (I): 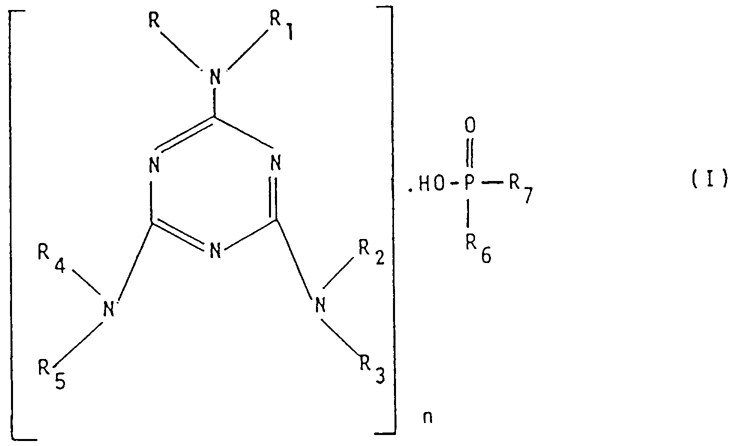 worin wenigstens eine der Gruppen R und R₁ bis R₅ ausgewählt ist aus -[-CₘH₂ₘ-]-O-R₈ und
-[-CₚH₂ₚ-]-N(R')₂
worin
m = eine ganze Zahl zwischen 2 und 8,
p = eine ganze Zahl zwischen 2 und 6,
R₈ = H, (C₁-C₈)-Alkyl, (C₂-C₆)-Alkenyl, -[-C_{q}H_{2q}-]-O-R₉, wobei
q eine ganze Zahl zwischen 1 und 4 und
R₉ Wasserstoff oder (C₁-C₄)-Alkyl, (C₆-C₁₂)-Cycloalkyl oder (C₆-C₁₂)-Alkylcycloalkyl bedeutet,
die Gruppen R', gleich oder voneinander verschieden, ausgewählt sind aus H, (C₁-C₈)-Alkyl, (C₂-C₆]-Alkenyl, (C₆-C₁₂)-Cycloalkyl oder (C₆-C₁₂)-Alkylcycloalkyl und (C₁-C₄)-Hydroxyalkyl, vorausgesetzt, daß die nachfolgend definierten Gruppen R₆ und R₇ ungleich H bzw. OH sind,
oder die Gruppe N(R')₂ durch einen Rest eines N-Heterocyclus ersetzt ist, der gegebenenfalls ein anderes Heteroatom enthält und an die Alkylkette durch das Stickstoffatom gebunden ist,
oder in der allgemeinen Formel (I) wenigstens eine der Gruppen NRR₁, NR₂R₃ und NR₄R₅ durch einen Rest eines N-Heterocyclus ersetzt ist, der gegebenenfalls ein anderes Heteroatom enthält und an den Triazinring durch das Stickstoffatom gebunden ist,
die restlichen Gruppen R und R₁ bis R₅, gleich oder voneinander verschieden, die oben angegebenen Bedeutungen haben oder ausgewählt sind aus H, (C₁-C₁₈)-Alkyl, (C₂-C₈)-Alkenyl, (C₆-C₁₆)-Cycloalkyl oder (C₆-C₁₆)-Alkylcycloalkyl, gegebenenfalls substituiert mit einer Hydroxy- oder (C₁-C₄)-Hydroxyalkylgruppe,
n eine Zahl zwischen 0.5 und 6 ist,
R₆ ausgewählt ist aus H, OH, (C₁-C₈)-Alkoxy, Aryloxy, gegebenenfalls substituiert durch eine (C₁-C₈)-Alkylgruppe, Aralkyl, gegebenenfalls substitiert mit einer (C₁₋C₄)-Alkylgruppe, (C₁-C₄)-Alkyl, gegebenenfalls substituiert mit einer Carboxylgruppe, und Aryl,
R₇ ausgewählt ist aus H, OH, (C₁-C₈)-Alkoxy, Aryloxy, (C₁-C₄)-Alkyl, Aryl, eine Gruppe der Formel 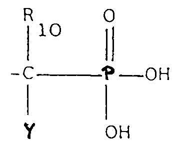
worin R₁₀ Wasserstoff oder (C₁-C₁₂)-Alkyl ist und Y OH oder R₁₀,
eine Gruppe der Formel 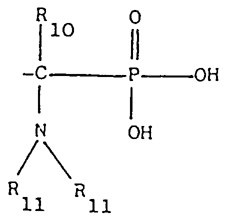 worin R₁₀ wie oben definiert ist und die Gruppen R₁₁, gleich oder verschieden voneinander, Wasserstoff oder (C₁-C₄)-Alkyl darstellen oder die Einheit N(R₁₁)₂ durch einen Rest eines N-Heterocyclus ersetzt ist, der gegebenenfalls ein anderes Heteroatom enthält und an das Kohlenstoffatom durch das Stickstoffatom gebunden ist,
eine Gruppe der Formel 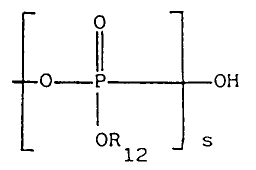 worin R₁₂ Wasserstoff oder (C₁-C₈)-Alkyl ist und s eine ganze Zahl von 1 bis 3,
eine Gruppe der Formel 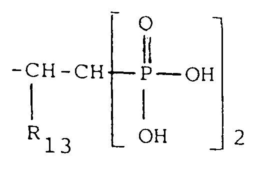 worin R₁₃ Wasserstoff oder Hydroxy ist,
eine Gruppe der Formel 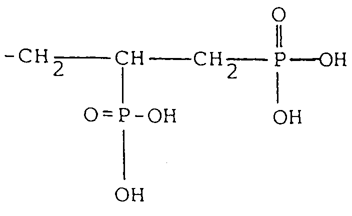 eine Gruppe der Formel 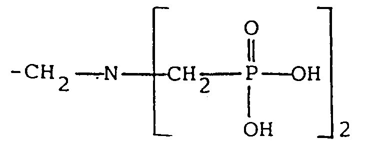 eine Gruppe der Formel 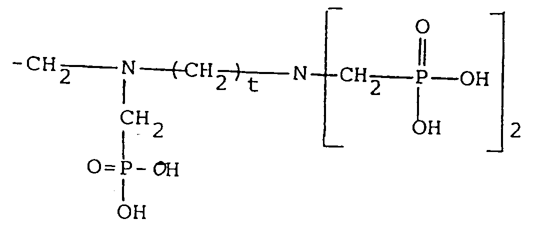 und eine Gruppe der Formel 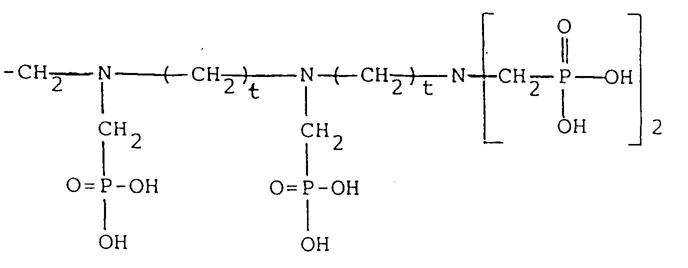 worin t eine ganze Zahl zwischen 2 und 6 ist;
oder R₆ und R₇ zusammen eine cyclische Struktur einer der folgenden Formeln bilden: 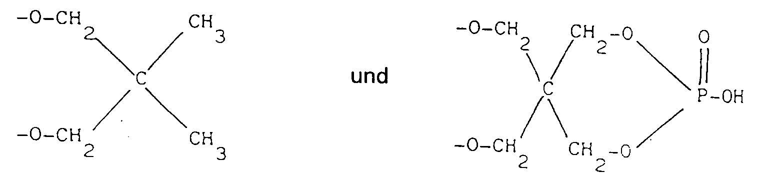 umfassend die Umsetzung von n Mol eines Derivats von 2,4,6-Triamino-1,3,5-triazin der allgemeinen Formel (II): 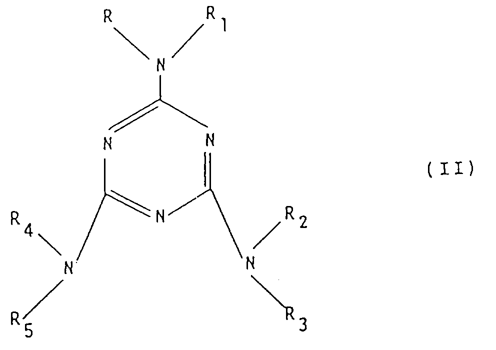 worin n und die Substituenten R und R₁ bis R₅ die oben angegebenen Bedeutungen haben, mit 1 Mol einer phosphorhaltigen Säure der allgemeinen Formel (III): 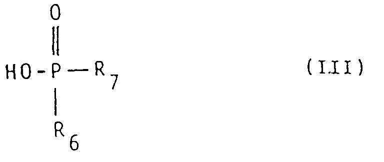 worin R₆ und R₇ die oben genannten Bedeutungen besitzen.

2. Verfahren gemäß Anspruch 1, worin eine oder mehrere der Einheiten NRR₁, NR₂R₃ und NR₄R₅ in der allgemeinen Formel (I) heterocyclische Reste darstellen, die ausgewählt sind aus Aziridinyl, Pyrrolidinyl, Piperidinyl, Morpholinyl, Thiomorpholinyl, Piperazinyl, 4-Methylpiperazinyl, 4-Ethylpiperazinyl, 2-Methylpiperazinyl, 2,5-Dimethylpiperazinyl, 2,3,5,6-Tetramethylpiperazinyl, 2,2,5,5-Tetramethylpiperazinyl, 2-Ethylpiperazinyl und 2,5-Diethylpiperazinyl.

3. Verfahren gemäß irgendeinem der Ansprüche 1 und 2, worin die Einheit N(R')₂ einen heterocyclischen Rest darstellt, der ausgewählt ist aus Aziridinyl, Pyrrolidinyl, Piperidinyl, Morpholinyl, Thiomorpholinyl, Piperazinyl, 4-Methylpiperazinyl und 4-Ethylpiperazinyl.

4. Verfahren gemäß irgendeinem der Ansprüche 1 bis 3, worin die Säure des Phosphors ausgewählt ist aus Hypophosphorsäure, Phosphoriger Säure, Phosphorsäure, Pyrophosphorsäure, Tripolyphosphorsäure, Ethan-1,1,2-triphosphonsäure, 2-Hydroxyethan-1,1,2-triphosphonsäure, Propan-1,2,3-triphosphonsäure, Isopropylphosphorsäure, n-Butylphosphorsäure, n-Butylphosphorsäure, Di-n-butylphosphorsäure, Diisopropylphosphorsäure, Di-n-pentylphosphorsäure, Isooctylphosphorsäure, Ethylphosphorsäure, Hexylphosphorsäure, 2-Ethylhexylphosphorsäure, Methylphosphonsäure, Ethylphosphonsäure, n-Propylphosphonsäure, n-Butylphosphonsäure, Aminomethylphosphonsäure, Phenylphosphorsäure, Phenylphosphonsäure, Phenylphosphinsäure, Di-n-butylpyrophosphorsäure, Di(2-ethylhexyl)pyrophosphorsäure, Octylphenylphosphorsäure, 2-Methylbenzylphosphonsäure, 1-Aminoethan-1,1-diphosphonsäure, 1-Hydroxyethan-1,1-diphosphonsäure, 1-Hydroxydodecan-1,1-diphosphonsäure,1-(N-Methylamino)ethan-1,1-diphosphonsäure,N,N-Dimethylaminomethan-1,1-diphosphonsäure, N-Butylaminomethan-1,1-diphosphonsäure, Phosphonoessigsäure, 2-Phosphonopropionsäure, 3-Phosphonopropionsäure, 2-Phosphonobuttersäure, 4-Phosphonobuttersäure, 2-Hydroxy-5,5-dimethyl-2-oxo-1,3,2-dioxophosphorinan, 3,9-Dihydroxy-2,4,8,10-tetroxo-3,9-diphosphaspiro[5.5]undecan-3,9-dioxid,Amino-tris(methylenphosphon)säure, Ethylendiaminotetra(methylenphosphon)säure, Hexamethylendiaminotetra(methylenphosphon)säure und Diethylentriaminopenta(methylenphosphon)säure.

5. Verfahren gemäß irgendeinem der Ansprüche 1 bis 4, worin die Umsetzung zwischen den Derivaten der allgemeinen Formel (II) und den Säuren der allgemeinen Formel (III) in Gegenwart eines Lösemittels und bei einer Temperatur im Bereich von 0 °C bis zum Siedepunkt des verwendeten Lösemittels durchgeführt wird.

6. Salze der Triazinderivate mit sauerstoffhaltigen Säuren des Phosphors mit der allgemeinen Formel (I), wie in Anspruch 1 definiert.

## Revendications (Revendications pour l'(les) Etat(s) contractant(s) suivant(s): AT, BE, CH, DE, DK, FR, GB, LI, NL, SE)

1. Sels de dérivés de la triazine avec des acides oxygénés dérivés du phosphore, répondant à la formule générale (I) : 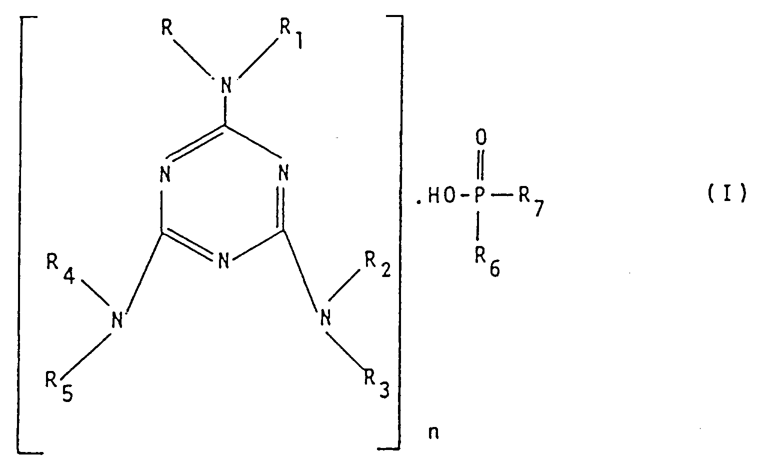 dans laquelle au moins l'un des groupes R et R₁ à R₅ est choisi parmi les groupes de formules :
⁅CₘH₂ₘ⁆O-R₈ et 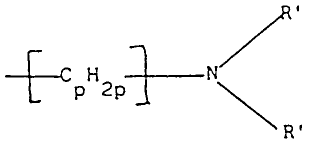 dans lesquelles :
m représente un nombre entier égal à 2 à 8,
p représente un nombre entier égal à 2 à 6,
R₈ représente un atome d'hydrogène, un groupe alkyle en C₁ à C₈, un groupe alcényle en C₂ à C₆ ou un groupe de formule ⁅C_{q}H_{2q}⁆O-R₉, dans laquelle :
q représente un nombre entier égal à 1 à 4, et
R₉ représente un atome d'hydrogène, un groupe alkyle en C₁ à C₄, un groupe cycloalkyle en C₆ à C₁₂ ou un groupe alkylcycloalkyle en C₆ à C₁₂,
les groupes R' sont identiques ou différents et représentent chacun un atome d'hydrogène, un groupe alkyle en C₁ à C₈, un groupe alcényle en C₂ à C₆, un groupe cycloalkyle en C₆ à C₁₂ ou un groupe alkylcycloalkyle en C₆ à C₁₂, ou bien un groupe hydroxyalkyle en C₁ à C₄, à la condition que les groupes R₆ et R₇ indiqués ci-après, soient différents d'un atome d'hydrogène et d'un groupe OH, respectivement,
ou bien le fragment N(R')₂ est remplacé par un radical N-hétérocyclique qui contient éventuellement un autre hétéroatome et qui est lié à la chaîne alkyle par l'intermédiaire de l'atome d'azote,
ou bien, dans la formule générale (I), au moins l'un des groupes NRR₁, NR₂R₃ et NR₄R₅ est remplacé par un radical N-hétérocyclique qui contient éventuellement un autre hétéroatome et qui est lié au cycle de la triazine par l'intermédiaire de l'atome d'azote,
les groupes R et R₁ à R₅ restants sont identiques ou différents et ont les significations indiquées précédemment ou sont choisis parmi l'atome d'hydrogène, les groupes alkyle en C₁ à C₁₈, les groupes alcényle en C₂ à C₈, les groupes cycloalkyle ou alkylcycloalkyle en C₆ à C₁₆, éventuellement substitués par un groupe hydroxy, et les groupes hydroxyalkyle en C₁ à C₄,
n représente un nombre de 0,5 à 6,
R₆ représente un atome d'hydrogène ou un groupe OH, alcoxy en C₁ à C₈, aryloxy, éventuellement substitué par un groupe alkyle en C₁ à C₈, aralkyle, éventuellement substitué par un groupe alkyle en C₁ à C₄, alkyle en C₁ à C₄, éventuellement substitué par un groupe carboxylique, ou aryle,
R₇ représente un atome d'hydrogène ou un groupe OH, alcoxy en C₁ à C₈, aryloxy, alkyle en C₁ à C₄ ou aryle, ou un groupe de formule : 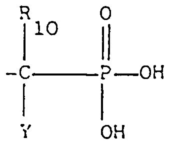 dans laquelle R₁₀ représente un atome d'hydrogène ou un groupe alkyle en C₁ à C₁₂ et Y représente un groupe OH ou R₁₀,
un groupe de formule : 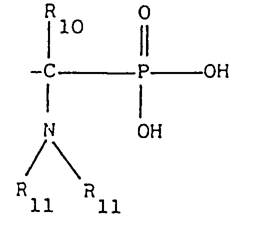 dans laquelle R₁₀ est tel que défini précédemment et les groupes R₁₁ sont identiques ou différents et représentent chacun un atome d'hydrogène ou un groupe alkyle en C₁ à C₄, ou bien le fragment N(R₁₁)₂ est remplacé par un radical N-hétérocyclique qui contient éventuellement un autre hétéroatome et qui est lié à l'atome de carbone par l'intermédiaire de l'atome d'azote,
un groupe de formule : 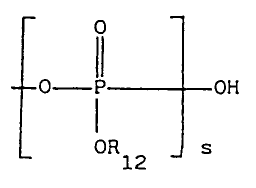 dans laquelle R₁₂ représente un atome d'hydrogène ou un groupe alkyle en C₁ à C₈ et s représente un nombre entier égal à 1 à 3,
un groupe de formule : 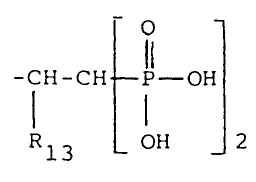 dans laquelle R₁₃ représente un atome d'hydrogène ou un groupe hydroxy,
un groupe de formule : 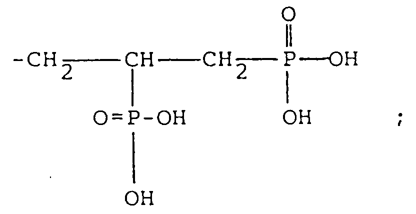 un groupe de formule : 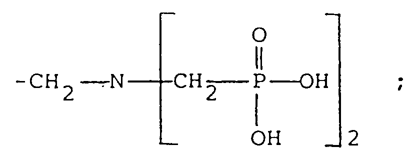 un groupe de formule : 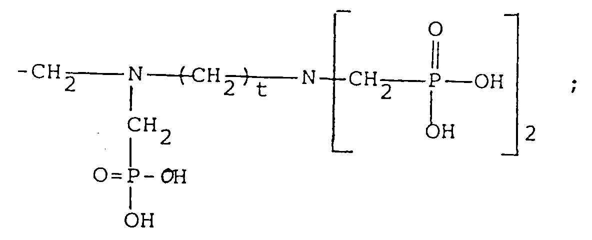 ou un groupe de formule : 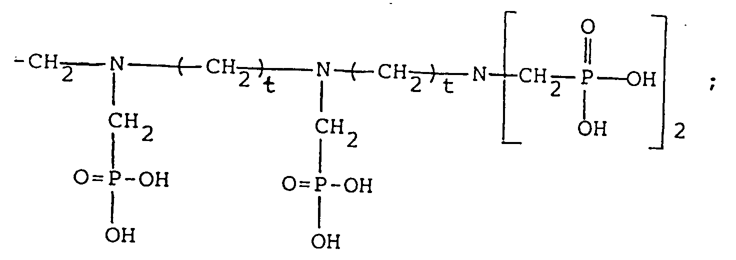 dans laquelle t représente un nombre entier égal à 2 à 6,
ou bien R₆ et R₇ forment ensemble une structure cyclique correspondant à l'une ou l'autre des formules suivantes :

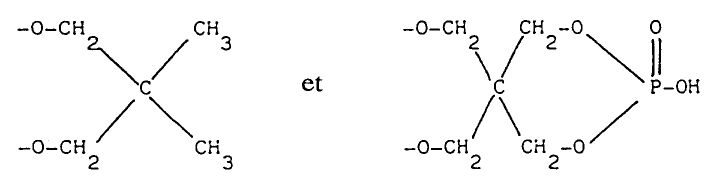

2. Sels selon la revendication 1, dans lesquels un ou plusieurs des fragments NRR₁, NR₂R₃ et NR₄R₅ de la formule générale (I) représentent un radical hétérocyclique choisi parmi les groupes aziridinyle, pyrrolidinyle, pipéridinyle, morpholinyle, thiomorpholinyle, pipérazinyle, 4-méthylpipérazinyle, 4-éthylpipérazinyle, 2-méthylpipérazinyle, 2,5-diméthylpipérazinyle, 2,3,5,6-tétraméthylpipérazinyle, 2,2,5,5-tétraméthylpipérazinyle, 2-éthylpipérazinyle, et 2,5-diéthylpipérazinyle.

3. Sels selon l'une quelconque des revendications 1 et 2, dans lesquels le fragment N(R')₂ représente un radical hétérocyclique choisi parmi les groupes aziridinyle, pyrrolidinyle, pipéridinyle, morpholinyle, thiomorpholinyle, pipérazinyle, 4-méthylpipérazinyle, et 4-éthylpipérazinyle.

4. Sels selon l'une quelconque des revendications 1 à 3, dans lesquels l'acide dérivé du phosphore est choisi parmi l'acide hypophosphoreux, l'acide phosphoreux, l'acide phosphorique, l'acide pyrophosphorique, l'acide tripolyphosphorique, l'acide éthane-1,1,2-triphosphonique, l'acide 2-hydroxyéthane-1,1,2-triphosphonique, l'acide propane-1,2,3-triphosphonique, l'acide isopropylphosphorique, l'acide n-butylphosphorique, l'acide di(n-butyl)phosphorique, l'acide diisopropylphosphorique, l'acide di(n-pentyl)phosphorique, l'acide isooctylphosphorique, l'acide éthylphosphorique, l'acide hexylphosphorique, l'acide 2-éthylhexylphosphorique, l'acide méthylphosphonique, l'acide éthylphosphonique, l'acide n-propylphosphonique, l'acide n-butylphosphonique, l'acide aminométhylphosphonique, l'acide phénylphosphorique, l'acide phénylphosphonique, l'acide phénylphosphinique, l'acide di(n-butyl)pyrophosphorique, l'acide di(2-éthylhexyl)pyrophosphorique, l'acide octylphénylphosphorique, l'acide 2-méthylbenzylphosphonique, l'acide 1-aminoéthane-1,1-diphosphonique, l'acide 1-hydroxyéthane-1,1-diphosphonique, l'acide 1-hydroxydodécane-1,1-diphosphonique, l'acide 1-(N-méthylamino)éthane-1,1-diphosphonique, l'acide N,N-diméthylaminométhane-1,1-diphosphonique, l'acide N-butylaminométhane-1,1-diphosphorique, l'acide phosphonoacétique, l'acide 2-phosphonopropionique, l'acide 3-phosphonopropionique, l'acide 2-phosphonobutyrique, l'acide 4-phosphonobutyrique, le 2-hydroxy-5,5-diméthyl-2-oxo-1,3,2-dioxophosphorinane, le 3,9-dihydroxy-2,4,8,10-tétroxo-3,9-diphosphaspiro[5.5] undécane-3,9-dioxyde, l'acide aminotris(méthylènephosphonique), l'acide éthylènediaminotétra(méthylènephosphonique), l'acide hexaméthylènediaminotétra(méthylènephosphonique), et l'acide diéthylènetriaminopenta(méthylènephosphonique).

5. Procédé de préparation des sels de formule générale (I) selon l'une quelconque des revendications 1 à 4, qui comprend la réaction de n mole d'un dérivé de la 2,4,6-triamino-1,3,5-triazine, de formule générale (II) : 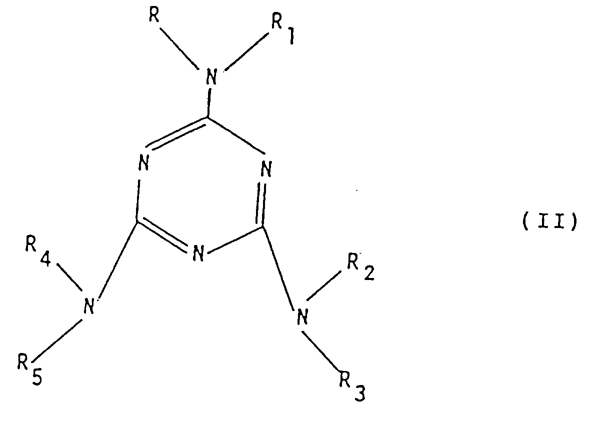 dans laquelle les substituants R et R₁ à R₅ ont les significations indiquées à la revendication 1, et n étant tel que défini à la revendication 1, avec 1 mole d'un acide contenant du phosphore, de formule générale (III) : 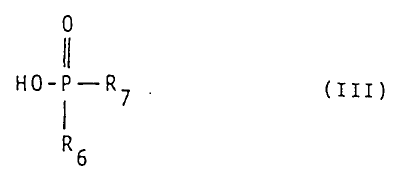 dans laquelle R₆ et R₇ ont les significations indiquées à la revendication 1.

6. Procédé selon la revendication 5, dans lequel la réaction entre les dérivés de formule générale (II) et les acides de formule générale (III) est effectuée en présence d'un solvant, à une température comprise dans l'intervalle allant de 0°C à la temperature d'ébullition du solvant utilisé.

## Revendications (Revendications pour l'(les) Etat(s) contractant(s) suivant(s): ES)

1. Procédé de préparation de sels de dérivés de la triazine avec des acides oxygénés dérivés du phosphore, répondant à la formule générale (I) : 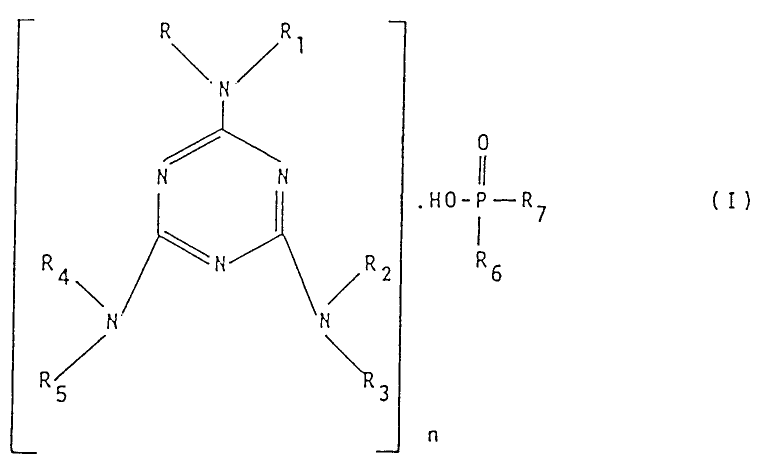 dans laquelle au moins l'un des groupes R et R₁ à R₅ est choisi parmi les groupes de formules :
⁅CₘH₂ₘ⁆O-R₈ et 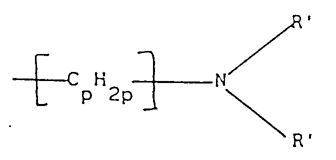 dans lesquelles :
m représente un nombre entier égal à 2 à 8,
p représente un nombre entier égal à 2 à 6,
R₈ représente un atome d'hydrogène, un groupe alkyle en C₁ à C₈, un groupe alcényle en C₂ à C₆ ou un groupe de formule ⁅C_{q}H_{2q}⁆O-R₉, dans laquelle :
q représente un nombre entier égal à 1 à 4, et
R₉ représente un atome d'hydrogène, un groupe alkyle en C₁ à C₄, un groupe cycloalkyle en C₆ à C₁₂ ou un groupe alkylcycloalkyle en C₆ à C₁₂,
les groupes R' sont identiques ou différents et représentent chacun un atome d'hydrogène, un groupe alkyle en C₁ à C₈, un groupe alcényle en C₂ à C₆, un groupe cycloalkyle en C₆ à C₁₂ ou un groupe alkylcycloalkyle en C₆ à C₁₂, ou bien un groupe hydroxyalkyle en C₁ à C₄, à la condition que les groupes R₆ et R₇ indiqués ci-après, soient différents d'un atome d'hydrogène et d'un groupe OH, respectivement,
ou bien le fragment N(R')₂ est remplacé par un radical N-hétérocyclique qui contient éventuellement un autre hétéroatome et qui est lié à la chaîne alkyle par l'intermédiaire de l'atome d'azote,
ou bien, dans la formule générale (I), au moins l'un des groupes NRR₁, NR₂R₃ et NR₄R₅ est remplacé par un radical N-hétérocyclique qui contient éventuellement un autre hétéroatome et qui est lié au cycle de la triazine par l'intermédiaire de l'atome d'azote,
les groupes R et R₁ à R₅ restants sont identiques ou différents et ont les significations indiquées précédemment ou sont choisis parmi l'atome d'hydrogène, les groupes alkyle en C₁ à C₁₈, les groupes alcényle en C₂ à C₈, les groupes cycloalkyle ou alkylcycloalkyle en C₆ à C₁₆, éventuellement substitués par un groupe hydroxy, et les groupes hydroxyalkyle en C₁ à C₄,
n représente un nombre de 0,5 à 6,
R₆ représente un atome d'hydrogène ou un groupe OH, alcoxy en C₁ à C₈, aryloxy, éventuellement substitué par un groupe alkyle en C₁ à C₈, aralkyle, éventuellement substitué par un groupe alkyle en C₁ à C₄, alkyle en C₁ à C₄, éventuellement substitué par un groupe carboxylique, ou aryle,
R₇ représente un atome d'hydrogène ou un groupe OH, alcoxy en C₁ à C₈, aryloxy, alkyle en C₁ à C₄ ou aryle, ou un groupe de formule : 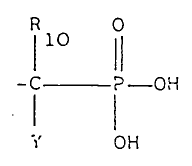 dans laquelle R₁₀ représente un atome d'hydrogène ou un groupe alkyle en C₁ à C₁₂ et Y représente un groupe OH ou R₁₀,
un groupe de formule : 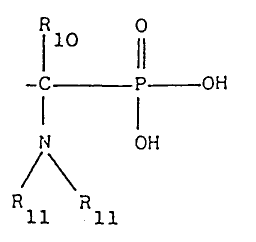 dans laquelle R₁₀ est tel que défini précédemment et les groupes R₁₁ sont identiques ou différents et représentent chacun un atome d'hydrogène ou un groupe alkyle en C₁ à C₄, ou bien le fragment N(R₁₁)₂ est remplacé par un radical N-hétérocyclique qui contient éventuellement un autre hétéroatome et qui est lié à l'atome de carbone par l'intermédiaire de l'atome d'azote,
un groupe de formule : 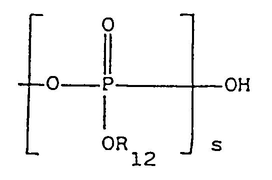 dans laquelle R₁₂ représente un atome d'hydrogène ou un groupe alkyle en C₁ à C₈ et s représente un nombre entier égal à 1 à 3,
un groupe de formule : 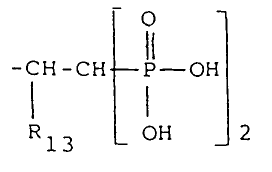 dans laquelle R₁₃ représente un atome d'hydrogène ou un groupe hydroxy,
un groupe de formule : 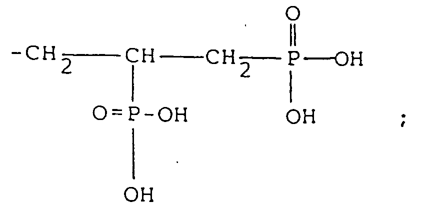 un groupe de formule : 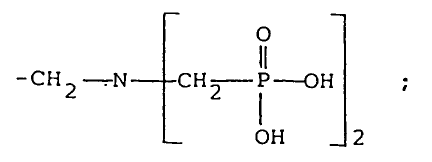 un groupe de formule : 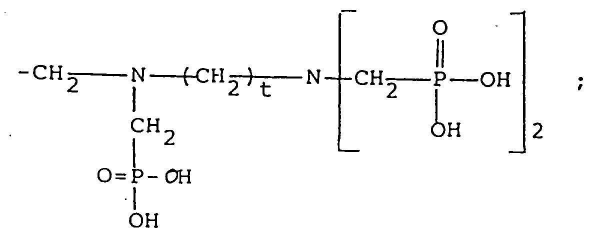 ou un groupe de formule : 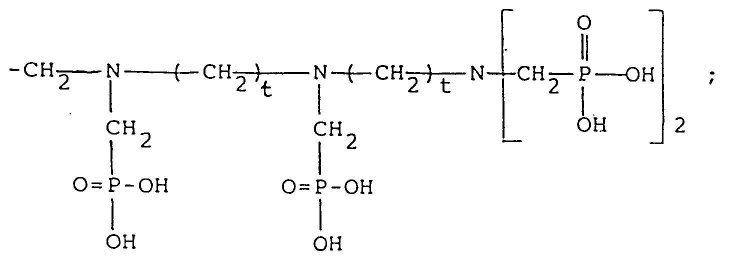 dans laquelle t représente un nombre entier égal à 2 à 6,
ou bien R₆ et R₇ forment ensemble une structure cyclique correspondant à l'une ou l'autre des formules suivantes : 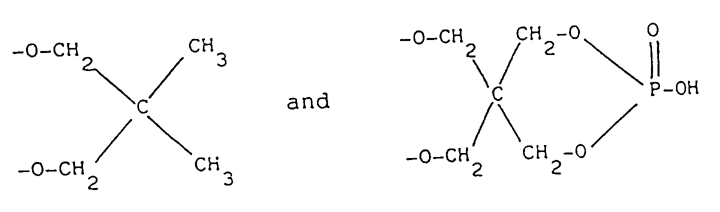 procédé qui comprend la réaction de n mole d'un dérivé de la 2,4,6-triamino-1,3,5-triazine, de formule générale (II) : 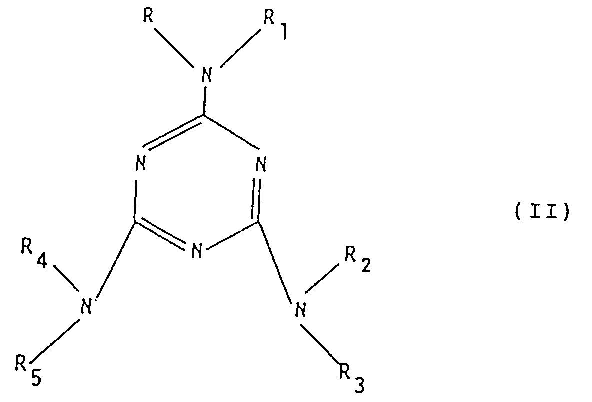 dans laquelle les substituants R et R₁ à R₅ ont les significations indiquées précédemment et n étant tel que défini précédemment, avec 1 mole d'un acide contenant du phosphore, de formule générale (III) : 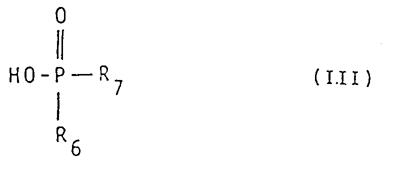 dans laquelle R₆ et R₇ ont les significations indiquées précédemment.

2. Procédé selon la revendication 1, dans lequel un ou plusieurs des fragments NRR₁, NR₂R₃ et NR₄R₅ de la formule générale (I) représentent un radical hétérocyclique choisi parmi les groupes aziridinyle, pyrrolidinyle, pipéridinyle, morpholinyle, thiomorpholinyle, pipérazinyle, 4-méthylpipérazinyle, 4-éthylpipérazinyle, 2-méthylpipérazinyle, 2,5-diméthylpipérazinyle, 2,3,5,6-tétraméthylpipérazinyle, 2,2,5,5-tétraméthylpipérazinyle, 2-éthylpipérazinyle, et 2,5-diéthylpipérazinyle.

3. Procédé selon l'une quelconque des revendications 1 et 2, dans lequel le fragment N(R')₂ représente un radical hétérocyclique choisi parmi les groupes aziridinyle, pyrrolidinyle, pipéridinyle, morpholinyle, thiomorpholinyle, pipérazinyle, 4-méthylpipérazinyle, et 4-éthylpipérazinyle.

4. Procédé selon l'une quelconque des revendications 1 à 3, dans lequel l'acide dérivé du phosphore est choisi parmi l'acide hypophosphoreux, l'acide phosphoreux, l'acide phosphorique, l'acide pyrophosphorique, l'acide tripolyphosphorique, l'acide éthane-1,1,2-triphosphonique, l'acide 2-hydroxyéthane 1,1,2-triphosphonique, l'acide propane-1,2,3-triphosphonique, l'acide isopropylphosphorique, l'acide n-butylphosphorique, l'acide di(n-butyl)phosphorique, l'acide diisopropylphosphorique, l'acide di(n-pentyl)phosphorique, l'acide isooctylphosphorique, l'acide éthylphosphorique, l'acide hexylphosphorique, l'acide 2-éthylhexylphosphorique, l'acide méthylphosphonique, l'acide éthylphosphonique, l'acide n-propylphosphonique, l'acide n-butylphosphonique, l'acide aminométhylphosphonique, l'acide phénylphosphorique, l'acide phénylphosphonique, l'acide phénylphosphinique, l'acide di(n-butyl)pyrophosphorique, l'acide di(2-éthylhexyl)pyrophosphorique, l'acide octylphénylphosphorique, l'acide 2-méthylbenzylphosphonique, l'acide 1-aminoéthane-1,1-diphosphonique, l'acide 1-hydroxyéthane-1,1-diphosphonique, l'acide 1-hydroxydodécane-1,1-diphosphonique, l'acide 1-(N-méthylamino)éthane-1,1-diphosphonique, l'acide N,N-diméthylaminométhane-1,1-diphosphonique, l'acide N-butylaminométhane-1,1-diphosphonique, l'acide phosphonoacétique, l'acide 2-phosphonopropionique, l'acide 3-phosphonopropionique, l'acide 2-phosphonobutyrique, l'acide 4-phosphonobutyrique, le 2-hydroxy-5,5-diméthyl-2-oxo-1,3,2-dioxophosphorinane, le 3,9-dihydroxy-2,4,8,10-tétroxo-3,9-diphosphaspiro[5.5] undécane-3,9-dioxyde, l'acide aminotris(méthylènephosphonique), l'acide éthylènediaminotétra(méthylènephosphonique), l'acide hexaméthylènediaminotétra(méthylènephosphonique), et l'acide diéthylènetriaminopenta(méthylènephosphonique).

5. Procédé selon l'une quelconque des revendications 1 à 4, dans lequel la réaction entre les dérivés de formule générale (II) et les acides de formule générale (III) est effectuée en présence d'un solvant, à une température comprise dans l'intervalle allant de 0°C à la température d'ébullition du solvant utilisé.

6. Sels de dérivés de la triazine avec des acides oxygénés dérivés du phosphore, répondant à la formule générale (I) telle que définie à la revendication 1 .
